# EUROPEAN PATENT APPLICATION

(11) **EP 4 404 123 A1**
(43) Date of publication of application: **24.07.2024**
(21) Application number: 22870215.5
(22) Date of filing: 08.09.2022
(51) Int. Cl.: G06Q 10/10, G06F 40/166, G06F 40/103, G16H 10/60, G16H 50/50, G16H 50/20, G16H 80/00, G16H 10/20

(54) **METHOD OF GENERATING INDIVIDUAL-DIRECTED INFORMATION RECORD SHEET BY MIR SERVER**

(30) Priority: 14.09.2021 KR 20210122668; 05.11.2021 KR 20210151544
(71) Applicant: Seegene, Inc., Seoul 05548 (KR)
(72) Inventor: CHUN, Jong Yoon, Yeoju-si Gyeonggi-do 12601 (KR)
(74) Representative: Viering, Jentschura & Partner mbB Patent- und Rechtsanwälte
(86) International application number: PCT/KR2022/013508
(87) International publication number: WO 2023/043130

(57) **Abstract**

The present invention relates to a method of generating an individual-directed information record sheet, and relates to a method of receiving time-series information input by an individual, and generating an information record sheet.

## Description

### [TECHNICAL FIELD]

The present disclosure relates to relates to a method of generating an individual-directed information record sheet and relates to a method of receiving time-series information input by an individual and generating an information record sheet.

### [BACKGROUND ART]

Recently, applications that record various aspects of an individual's daily life using mobile terminals have been used. Users may select an application to record information according to a topic of personal interest, install the application on their mobile terminals, and then use the installed application.

Therefore, if the topics of interest, such as healthcare, hobbies, daily life, or work, are different, all related applications should be installed. These multiple applications are configured to generate a single record and store the record on a mobile terminal or on a server.

However, each application only stores recorded data and has the problem of making it difficult for users to reuse or transmit information on the recorded data to other users. Of course, the recorded data may be shared and open to others, but it is not possible to recognize the history of the records, such as the order in which the recorded data was recorded and whether any modifications was made. In addition, it is difficult to infer what a drafter's intention was when he/she wrote it.

Even in the case of disclosing recorded data, if all contents including the recorded data should be disclosed while some data are not disclosed, there is a problem in that a corresponding part of the data should be deleted and re-shared.

In addition, recently, as the spread of personal portable terminals (so-called smartphones) has increased, devices and applications for self-recording individuals' health have been developed. One of them is a health record application that records an individual's health in various ways.

For example, health record applications allow measurement results from a device that measures an individual's blood pressure, blood sugar, heart rate, etc. to be automatically or manually input into an application on a mobile terminal. At this time, the application analyzes the measurement results and provides guidance on the analyzed results to the user of the mobile terminal. Guidance may include an alarm as to whether a measurement record falls within a normal or abnormal range. If the measurement record falls within the abnormal range, an alarm requesting a visit to a medical institution may be included.

As described above, current health record applications are linked to biometric measurement devices, record measured biometric information, and receive feedback on the biometric information.

Since these health records are stored through an application of individuals' mobile terminals, there is a problem in that it is difficult to recover the health record information due to loss or damage of mobile terminals.

### [DETAILED DESCRIPTION OF INVENTION]

### [TECHNICAL PROBLEMS]

According to an embodiment of the present disclosure, the present disclosure provides a method of operating a membership-led information record (MIR) server, which is a computing device that generates an individual-directed information record sheet using a MIR application installed on an individual's mobile terminal.

According to an embodiment of the present disclosure, the present disclosure provides a method of operating a MIR server that generates an individual-directed information record sheet in response to one category of interest selected from a plurality of categories of interest.

According to an embodiment of the present disclosure, the present disclosure provides a method of operating a MIR server that receives time-series information at least twice through a mobile terminal and generates an individual-directed information record sheet.

According to an embodiment of the present disclosure, the present disclosure provides a method of generating a MIR server that provides a terminal environment-controlling instruction to a mobile terminal to allow time-series information to be input in response to one category of interest selected from a plurality of categories of interest.

According to an embodiment of the present disclosure, the present disclosure provides a method of operating a MIR server that may provide a process (history) of generating an individual-directed information record sheet.

According to an embodiment of the present disclosure, the present disclosure provides a method of operating a MIR server to perform a service that allows other users to search and read an individual-directed information record sheet shared in the MIR server.

According to an embodiment of the present disclosure, the present disclosure provides a method of operating a MIR server to perform a service that provides statistical data for a plurality of individual-directed information record sheets according to a user's request.

According to an embodiment of the present disclosure, the present disclosure provides a method of operating a MIR server so that a user may generate his own healthcare records using a terminal and generate a searchable individual-directed information record sheet using the generated healthcare records.

According to an embodiment of the present disclosure, the present disclosure provides a method of operating a MIR server that allows a user to input healthcare records, such as pre-visiting medical institution information, medical examination and treatment information provided by the medical institution, and post-medical treatment information for a treatment using a terminal in a MIR server and provides the healthcare records to the MIR server to generate an individual-directed information record sheet.

According to an embodiment of the present disclosure, the present disclosure provides a method of operating a MIR server to provide a service that allows a user to provide healthcare records by himself/herself and generate an individual-directed information record sheet whenever symptoms occur in the user, thereby enabling the user to generate, store, manage, and read individual-directed information record sheet regarding diseases that occur throughout his or her lifetime.

According to an embodiment of the present disclosure, the present disclosure provides a search service that generates healthcare records input by multiple users on a MIR server as each individual-directed information record sheet and stores the same in a search database (DB), thereby enabling general users who wish to obtain information on a treatment according to symptoms or diseases.

According to an embodiment of the present disclosure, the present disclosure provides a medical treatment appointment service that selects a medical category according to symptoms and/or health questionnaires in healthcare records input by a user and provides a list of medical institutions matching the selected medical category to a user terminal, so that a medical treatment appointment may be made to a medical institution selected by the user.

According to an embodiment of the present disclosure, the present disclosure provides a method of operating a MIR server that provides a service generating an individual-directed information record sheet using healthcare records recorded by a user and that also generates more accurate individual-directed information record sheet by further including personal health record (PHR) information held by a government/public institution in each individual-directed information record sheet.

According to an embodiment of the present disclosure, the present disclosure provides a search service that allows healthcare records input by multiple users on the MIR server to be generated as individual-directed information record sheets and stored in a search database, so that medical professionals at general medical institutions may collect information on medical treatment and/or treatment at other medical institutions for symptoms or diseases.

### [TECHNICAL SOLUTION]

In accordance with an aspect of the present disclosure, there is provided a method of generating a member information record sheet to be performed by a membership-led information record (MIR) server, the method comprises: (a) receiving, by the MIR server, log on information from a terminal of a membershiped information provider in which a MIR application is executed and validating the membershiped information provider, wherein the membershiped information provider is registered as a member in the MIR application; (b) receiving a category of interest corresponding to a topic of interest of the membershiped information provider; (c) providing a terminal environment-controlling instruction to the terminal of the membershiped information provider to input information on the topic of interest of the membershiped information provider, wherein the terminal environment-controlling instruction includes an information editing module, an information sharing-range setting module, and an information record sheet that is used in the terminal of the membershiped information provider; (d) receiving time-series information, which is information on the topic of interest input through the MIR application, in a time-series manner, wherein the time-series information is generated by inputting information on the topic of interest at least twice at time intervals; and (e) generating MIR using the time series information, wherein the MIR is tracked by a tracking module of the MIR server.

In accordance with another aspect of the present disclosure, there is provided a computing device for generating a membershiped information record sheet in a membership-led information record (MIR) server, the computing device comprises: a memory configured to store at least one instruction; and a processor configured to execute the one or more instructions stored in the memory, wherein the instructions, when executed by the processor, cause the processor to: (a) receive, by the MIR server, log on information from a terminal of a membershiped information provider in which a MIR application is executed and validating the membershiped information provider, wherein the membershiped information provider is registered as a member in the MIR application; (b) receive a category of interest corresponding to a topic of interest of the membershiped information provider; (c) provide a terminal environment-controlling instruction to the terminal of the membershiped information provider to input information on the topic of interest of the membershiped information provider, wherein the terminal environment-controlling instruction includes an information editing module, an information sharing-range setting module, and an information record sheet that is used in the terminal of the membershiped information provider; (d) receive time-series information, which is information on the topic of interest input through the MIR application, in a time-series manner, wherein the time-series information is generated by inputting information on the topic of interest at least twice at time intervals; and (e) generate MIR using the time series information, wherein the MIR is tracked by a tracking module of the MIR server.

In accordance with another aspect of the present disclosure, there is provided a non-transitory computer readable storage medium storing computer executable instructions, wherein the instructions, when executed by a processor, cause the processor to perform a method for generating a membershiped information record sheet in a membership-led information record (MIR) server, the terminal control method comprise: (a) receiving, by the MIR server, log on information from a terminal of a membershiped information provider in which a MIR application is run and validate the membershiped information provider, wherein the membershiped information provider is registered as a member in the MIR application; (b) receiving a category of interest corresponding to a topic of interest of the membershiped information provider; (c) providing a terminal environment-controlling instruction to the terminal of the membershiped information provider to input information on the topic of interest of the membershiped information provider, wherein the terminal environment-controlling instruction includes an information editing module, an information sharing-range setting module, and an information record sheet that is used in the terminal of the membershiped information provider; (d) receiving time-series information, which is information on the topic of interest input through the MIR application, in a time-series manner, wherein the time-series information is generated by inputting information on the topic of interest at least twice at time intervals; and (e) generating MIR using the time series information, wherein the MIR is tracked by a tracking module of the MIR server.

In accordance with another aspect of the present disclosure, there is provided a computer program stored in a non-transitory computer readable storage medium storing computer executable instructions, wherein the instructions, when executed by a processor, cause the processor to perform a terminal control method in a membership-led information record (MIR) server, the terminal control method comprise: (a) receiving, by the MIR server, log on information from a terminal of a membershiped information provider in which a MIR application is executed and validating the membershiped information provider, wherein the membershiped information provider is registered as a member in the MIR application; (b) receiving a category of interest corresponding to a topic of interest of the membershiped information provider; (c) providing a terminal environment-controlling instruction to the terminal of the membershiped information provider to input information on the topic of interest of the membershiped information provider, wherein the terminal environment-controlling instruction includes an information editing module, an information sharing-range setting module, and an information record sheet that is used in the terminal of the membershiped information provider; (d) receiving time-series information, which is information on the topic of interest input through the MIR application, in a time-series manner, wherein the time-series information is generated by inputting information on the topic of interest at least twice at time intervals; and (e) generating MIR using the time series information, wherein the MIR is tracked by a tracking module of the MIR server.

### [EFFECT OF INVENTION]

The features and advantages of the present disclosure are summarized as follows.
(1) The method of generating an individual-directed information record sheet by a membership-led information record (MIR) server of the present disclosure has the effect of efficiently converting information records input by individuals into a database in the MIR server.
(2) In the method of generating an individual-directed information record sheet by a MIR server of the present disclosure, the MIR stored as a plurality of MIR editions in the MIR server may provide history of the stored MIR editions, information traceability may be provided, and information credibility may be provided.
(3) The method of generating an individual-directed information record sheet by a MIR server of the present disclosure has the characteristics of easy editing convenience for information on topics of interest input to a MIR application and easy processability of information.
(4) The method of generating an individual-directed information record sheet by a MIR server of the present disclosure facilitates the generation, change, deletion, disclosure, and sharing of information by granting control of information to an individual who generated the information record.
(5) The method of generating an individual-directed information record sheet by a MIR server of the present disclosure has the effect of facilitating data collection for building big data and facilitating statistical processing of the collected data by providing an information record sheet for receiving standardized, formal, and quantitative information to a MIR application.
(6) The method of generating an individual-directed information record sheet by a MIR server of the present disclosure has the effect of checking development history using flow in which information is recorded by allowing information on a plurality of information of interest to be received sequentially.
(7) The method of generating an individual-directed information record sheet by a MIR server of the present disclosure has the effect of easily recognizing the type of information using an edition, an input order, and contents of a content by allocating an edition to information on topics of interest input sequentially.
(8) In the system, method, and storage medium of the present disclosure, individual-directed information record sheets are generated using personal healthcare records directly input by a user and stored in a search DB, so that other users may search and check the individual-directed information record sheets.
(9) In the system, method, and storage medium of the present disclosure, an individual-directed information record sheet is generated using personal healthcare records directly input by a user, and when an individual-directed information record sheet generated by further including medical examination and treatment information provided by a medical institution is provided to other users, more professional information from the medical institution may be further included, thereby improving the reliability of the individual-directed information record sheet.
(10) In the system, method, and storage medium of the present disclosure, a user may allow other users to share personal healthcare records including post-medical treatment information according to medical treatment of a medical institution, so that the other users may be easily provided with information on diseases with the same or similar symptoms.
(11) In the system, method, and storage medium of the present disclosure, pre-visiting medical institution information directly input by an individual, health examination, and treatment information, post-medical treatment information, and the like, rather than healthcare records held by medical institutions and/or government agencies, are stored as an individual-directed information record sheet, so that when the same symptom and/or disease occurs, the user may search and check the stored individual-directed information record sheet to easily cope with the disease.
(12) In the system, method, and storage medium of the present disclosure, healthcare records input by a user regarding symptoms and/or diseases are generated as an individual-directed information record sheet and the user's individual-directed information record sheet is stored sequentially, so that the user may maintain the personal healthcare records for life and access the records at any time.
(13) In the system, method, and storage medium of the present disclosure, an individual-directed information record sheet may be generated using personal healthcare records directly input by the user and the generated individual-directed information record sheet is stored in a search DB so that it may be searched, thereby providing a service that allows professional medical personnel at general medical institutions to collect information on medical treatment and/or treatment at other medical institutions.

### [BRIEF DESCRIPTION OF THE DRAWING]

FIG. 1 is a configuration diagram illustrating an individual-directed information record sheet generating system according to the present disclosure.
FIG. 2 is an exemplary diagram illustrating an embodiment of a screen configuration for selecting a category of interest in a membership-led information record (MIR) application according to an implementation of the present disclosure.
FIG. 3 is an exemplary diagram illustrating an embodiment of a category of interest including hierarchical information levels according to an implementation of the present disclosure.
FIG. 4 is an exemplary diagram illustrating an embodiment of a screen configuration for outputting an information record sheet in a MIR application according to an implementation of the present disclosure.
FIG. 5 is an exemplary diagram illustrating an embodiment of an information editing module that may be included in an information record sheet according to an implementation of the present disclosure.
FIG. 6 is an exemplary diagram illustrating an embodiment of a screen configuration for outputting an index of a MIR edition generated using a MIR application according to an implementation of the present disclosure.
FIG. 7 is an exemplary diagram illustrating an embodiment in which a hierarchically configured edition is provided in a MIR server according to an implementation of the present disclosure.
FIG. 8 is an exemplary diagram illustrating an embodiment in which a MIR server allocates an edition to time-series information provided from a terminal of a membershiped information provider to generate an edition index according to an implementation of the present disclosure.
FIG. 9 is an exemplary diagram illustrating an embodiment of meta information included in an edition allocated by a MIR server according to an implementation of the present disclosure.
FIG. 10 is a configuration diagram illustrating an individual-directed information record sheet generating system according to another implementation of the present disclosure.
FIG. 11 is a flowchart illustrating a method of generating an individual-directed information record sheet according to another implementation of the present disclosure.
FIG. 12 is a flowchart for generating an individual-directed information record sheet according to another implementation of the present disclosure.
FIG. 13 is a flowchart for making a reservation for a medical institution in an individual-directed information record sheet generating system according to another implementation of the present disclosure.
FIG. 14 is a flowchart for including PHR information in an individual-directed information record sheet generating system according to another implementation of the present disclosure.
FIG. 15 is a flowchart illustrating a method of suggesting treatment at a medical institution according to users' symptoms in an individual-directed information record sheet generating system according to another implementation of the present disclosure.
FIG. 16 is an exemplary diagram illustrating how an individual-directed information record sheet is generated and used according to another implementation of the present disclosure.

### [BEST MODE FOR CARRYING OUT THE PRESENT DISCLOSURE]

Hereinafter, the present disclosure will be described in detail with respect to embodiments. The embodiments according to the present disclosure are intended to fully convey the present disclosure, and those having ordinary knowledge will appreciate that the scope of the present disclosure is not limited by the embodiments.

In addition, in describing the component elements of the present invention, terms such as first, second, A, B, (a), (b), (i), and (ii) may be used. These terms are only for distinguishing the component from other components, and the nature, turn, or order of the component is not limited by the term. If a component is described as being "connected" or "coupled" to another component, the component may be directly connected to another component, but it should be understood that another component between each component may be "connected" or "coupled".

As used herein, the term 'membershiped information provider' refers to a user who has joined as a member or associate member to a membership-led information record (MIR) server and/or MIR application. Or, the `membershiped information provider' is a user authorized to generate a MIR by providing information.

The term `MIR' used herein is a member-led information record generated by the MIR server upon receiving time-series information provided from a membershiped information provider. In this specification, the term `member-directed information record' may be used interchangeably with `individual-directed information record'.

The term 'terminal of the membershiped information provider' used herein refers to a personal terminal owned by a membershiped information provider and has the MIR application installed therein. As used herein, the term `user terminal' may include all communicationavailable terminals, such as mobile terminals, personal computers (PCs), and tablet PCs, but mobile terminals are preferably used. The membershiped information provider may input his/her own information or the information of a managed user through the terminal of the membershiped information provider. In this specification, the term 'terminal of the membershiped information provider' may be used interchangeably with a `user terminal'.

As used herein, the term 'user' refers to a membershiped information provider who inputs information records using the MIR application. When a user inputs a healthcare record according to the input information, the user, that is, the membershiped information provider, may be a patient with symptoms of a disease or a guardian of the patient. Therefore, the term 'user' refers to either the patient or the patient's guardian. In addition, if the subject of the information record is a minor, the membershiped information provider may be a guardian.

The term `individual-directed information record sheet generating system' used herein may include a MIR server and a search DB. The MIR server, a computing device that generates an individual-directed information record, is physically separated from the search DB, and the term referring to the MIR server and search DB together may be called an individual-directed information record sheet generating system. Meanwhile, it is not excluded that the MIR server performs the function of a search DB. In addition, it is desirable that the term `individual-directed information record sheet generating system' includes the MIR server. In addition, in this specification, the term `MIR server' may be used interchangeably with a `individual-directed information record sheet generating server'.

As used herein, the term `healthcare record' is a term used in an embodiment of information recording. `Healthcare record' is a record of a user's disease input by a membershiped information provider or medical institution and includes text, documents, and video (including images, videos, and audio files). For example, `healthcare records' may include i) pre-visiting medical institution information. Pre-visiting medical institution information is information on the symptoms the user perceives about a disease. Pre-visiting medical institution information may be data in a text format provided by the user for healthcare records. The pre-visiting medical institution information may include medical image information generated by a medical institution in which the user has been treated previously. The pre-visiting medical institution information is input to a terminal of the membershiped information provider and transmitted.

The pre-visiting medical institution information may include a health questionnaire for inputting or marking symptoms according to questions.

The health questionnaire may be provided from a dedicated application of the terminal of the membershiped information provider and/or from an individual-directed information record sheet generating server.

The health questionnaire is a question in a text form, and the membershiped information provider may record it in a text form on the terminal of the membershiped information provider.

The health questionnaire may be written in the form of selecting one or more of a plurality of expected answers being provided.

The health questionnaire may be filled in such that a shape of the body in a graphic form is output to the terminal of the membershiped information provider and the membershiped information provider selects a position in which the user's symptoms occur in the shape of the body.

The health questionnaire may be received through the terminal of the membershiped information provider while performing multiple steps for each symptom.

For example, `healthcare records' may include ii) medical examination and treatment information. Medical examination and treatment information may be input by the membershiped information provider using the terminal of the membershiped information provider and provided to the MIR server. Medical examination and treatment information includes the results of examination by a medical professional at a medical institution regarding the user's symptoms, and the medical examination and treatment information may further include the name of a disease, a therapeutic drug, and/or a treatment method.

At this time, the medical examination and treatment information may be provided from a medical institution terminal. When the medical examination and treatment information is provided from the medical institution terminal, in an implementation of the present disclosure, the medical institution terminal may directly provide medical examination and treatment information to the individual-directed information record sheet generating server.

In another implementation of the present disclosure, the medical institution terminal may provide the second medical examination and treatment information to the terminal of the membershiped information provider, and the terminal of the membershiped information provider may generate third medical examination and treatment information including the input first medical examination and treatment information and the second medical examination and treatment information provided from the medical institution and provide the generated third medical examination and treatment information to the individual-directed information record sheet generating server.

In this specification, medical examination and treatment information provided from medical institutions is used interchangeably with the term `medical treatment information.'

For example, `healthcare records' may include iii) medical institution information. Medical institution information may include a name of the medical institution that has treated the user, a medical subject, a location of the medical institution, etc., and, if necessary, may further include a name of a medical practitioner who treated the user. At this time, medical institution information may be provided from the terminal of the membershiped information provider or the medical institution terminal.

For example, `healthcare records' may include iv) post-medical treatment information. The post-medical treatment information is a result of treatment performed on the user according to the medical treatment of the medical institution, such as treatment, medication, surgery, and consultation. The post-medical treatment information may include an additionally input information, such as diet, exercise therapy, and lifestyle habits at the time of treatment, which the user performed separately from medical treatment at a medical institution. The post-medical treatment information is input to the terminal of the membershiped information provider.

The membershiped information provider may provide the result by inputting the result as `post-medical treatment information' according to treatment of the medical institution.

The post-medical treatment information may be additionally input at the medical institution terminal. For example, the user may visit a medical institution multiple times for medical treatment and treatment of a disease. While providing treatment for a user who visits multiple times, the medical institution may input opinions about the treatment results during the visit as post-medical treatment information.

The term `individual-directed information record sheet' used herein may include a healthcare record in which a plurality of pieces of information input and provided from the terminal of the membershiped information provider are gathered into one. Here, the plurality of pieces of information are healthcare records provided by a symptom or disease occurring to the user. Depending on the type of healthcare records provided from the terminal of the membershiped information provider, the `healthcare record sheet' is generated to include text, documents, images (including files, such as images and videos), sound (including audio files, such as voices), etc.

The term `medical examination and treatment information' used herein includes the results of examination by a medical professional at a medical institution regarding the user's symptoms or disease. `Medical examination and treatment information' may include information, such as the name of the disease, disease code, characteristics of the disease, therapeutic drugs for the disease, and treatment method for the disease. `Medical examination and treatment information' may be input through the terminal of the membershiped information provider, but may be additionally provided from the terminal of the medical institution if necessary. At this time, `medical examination and treatment information' may be provided directly from the terminal of the medical institution to the individual-directed information record sheet generating server, or may be provided from the terminal of the medical institution to the terminal of the membershiped information provider and may be provided from the terminal of the membershiped information provider to the individual-directed information record sheet generating server.

The term `post-medical treatment information' used herein refers to information that is directly input to the terminal of the membershiped information provider about changes in the disease through treatment and/or prescribed therapeutic drug, treatment methods, etc. according to medical treatment.

`Post-medical treatment information' may include biometric information measured through a measurement device that measures the body according to symptoms and/or disease.

In an embodiment of the present disclosure, biometric information may include information, such as blood pressure, blood sugar, heart rate, body temperature, weight, height, and pulse.

`Post-medical treatment information' may include a plurality of pieces of information recorded sequentially during a treatment period, such as the user's medical treatment, medication, etc., for the same symptom and/or disease.

In an embodiment of the present disclosure, since fractures, burns, etc. require a long treatment period, a plurality of pieces of post-medical treatment information determined by the user may be recorded during the treatment period.

In an embodiment of the present disclosure, `post-medical treatment information' may include information on eating habits, exercise information, administration information of therapeutic drugs, and other information used to help treat the disease in addition to the treatment of the user's disease.

The term `medical institution' used herein refers to an institution in which a medical professional provides medical treatment and/or treatment to users. `Medical institution' may be classified according to various criteria, and in the present disclosure, `medical institution' may be a medical institution in which a medical professions may perform medical treatment. In addition, `medical institution' is a medical institution having one or more medical subjects to treat multiple diseases.

The user may visit a medical institution for treatment, but if necessary, a medical professional from the medical institution may visit the user and provide treatment.

In addition, the medical treatment for the user performed at a medical institution may include remote medical treatment.

As used herein, the term `time series' refers to listing or indexing a series of certain facts (including but not limited to events, tasks, phenomena, states and data points) sequentially (including regular and irregular time intervals). Therefore, `time-series information' is information generated in a sequential manner at time intervals. In other words, data that is generated sequentially over time may be included in `time series information.' For example, when two or more data generated sequentially on a specific topic are interpreted as timedependent, the corresponding data may be referred to as `time-series information.'

In addition, the `time-series manner' is a method in which `time-series information' generated in a time-series manner is input or received in a time-series manner. In the `time series manner', the generated `time series information' may be input or received in real-time or non-real time. In the term `time series information', 'information' may include data of the same type (identical type) or data of a non-identical type.

The term 'tracking' used herein refers to providing information on the generation flow of MIR generated by receiving time-series information in a time-series manner. In other words, the MIR server may provide development history in which a specific MIR is generated at the request of a membershiped information provider or member user. For example, a membershiped information provider may provide first information, second information, and third information corresponding to a topic of interest to the MIR server sequentially. The MIR server generates the MIR of the membershiped information provider using the first, second, and third information that are sequentially input.

At this time, when a membershiped information provider or a member user tracks the generated MIR, the development history of each of the first information, second information, and third information may be generated and provided.

The development history provided by tracking includes:
(i) contents on a date and time each information is generated and/or received
(ii) contents on changes to each information, number of changes, date and time of change, etc.
(iii) MIR edition assignment records for each information
(iv) meta-information on the MIR edition of each information
(v) location information corresponding to each piece of information

Tracking may only provide information selected by the membershiped information provider or member user among the information included in the development history as described above.

In an implementation of the present disclosure, a method of generating a member information record sheet by a MIR server, which is a computing device, includes the following steps:
(a) receiving, by a MIR server, log on information from a terminal of a membershiped information provider in which a MIR application is run and validating the membershiped information provider, wherein the membershiped information provider is registered as a member in the MIR application; (b) receiving a category of interest corresponding to a topic of interest of the membershiped information provider; (c) providing a terminal environment-controlling instruction to the terminal of the membershiped information provider so that information on a topic of interest of the membershiped information provider is input, wherein the terminal environment-controlling instruction includes an information editing module, an information sharing-range setting module, and an information record sheet that may be used in the terminal of the membershiped information provider; (d) receiving time-series information, which is information on the topic of interest input through the MIR application, in a time-series manner, wherein the time-series information is generated by inputting information on the topic of interest at least twice at time intervals; and (e) generating MIR using the time series information, wherein the MIR may be tracked by a tracking module of the MIR server.

The MIR may be generated to include additional information that is further input one or more times at the time intervals after an intermediate MIR is generated using the input information on the topic of interest.

The MIR may be generated by (i) incorporating the additional information into the intermediate MIR; (ii) modifying the intermediate MIR with the additional information; (iii) generating another intermediate MIR using the additional information separate from the intermediate MIR and then using two intermediate MIRs to generate the MIR; and/or (iv) performing a combination of (i) and (ii) above.

In the tracking, the MIR server provides development history from a perspective of the time series manner.

The development history may include at least one history selected from the group consisting of a reception history of the time-series information, a change history of information on the topic of interest, and a creation history of the MIR.

The MIR may be an edited information record.

The edited information record may include a plurality of MIR editions in which an edition is assigned to each of the MIR editions.

In the tracking, the MIR server may provide development history in terms of the edition, and the development history may include the edition assignment history.

The MIR edition may include meta-information on the edition.

The MIR includes two or more MIR editions, and the two or more MIR editions may be selected by a MIR server or the membershiped information provider.

The information on the topic of interest may be generated as a MIR edition by assigning an edition according to a point in time at which it is received in the time-series manner.

When additional information on the topic of interest is input after creation of the MIR edition, the MIR server may (i) further include information on the topic of interest in the MIR edition; (ii) automatically generate a new MIR edition; or (iii) generate a new MIR edition in response to a request of the membershiped information provider.

The edition may be assigned in plurality, and the plurality of assigned editions may be structured hierarchically.

The MIR server may provide the membershiped information provider with the authority to both edit the MIR and determine an information sharing range of the MIR.

The editing may be performed using the information editing module, and the determining of the range of sharing information may be performed using the information sharing range setting module.

The category of interest may include a hierarchical information level having multiple levels in the topic of interest.

The MIR server receives the category of interest at the lowest level of the hierarchical information level.

The category of interest may include at least one selected from a group including at least two or more from the group consisting of healthcare, art, research, cooking, pets, animal husbandry, plant cultivation, travel, religion, and sports. The types of categories of interest are not limited to the groups listed above.

When the category of interest is healthcare, the MIR is a healthcare record of an user selected from the membershiped information provider and a patients managed by the membershiped information provider; and the MIR includes: (i) a MIR edition including, as pre-visiting medical institution information, information on symptoms of a disease, disorder, or condition; (ii) a MIR edition including, as medical examination and treatment information, information including result of medical examination, treatment, surgery, prescription medication and/or therapy done by a medical professional; and (iii) a MIR edition including, as post-medical treatment information, information on the user's progress after treatment.

The pre-visiting medical institution information, the medical examination and treatment information, and the post-medical treatment information are types of time-series information, the time-series information is generated by inputting information on the topic of interest three or more times at time intervals, one of the types of information input three or more times is the pre-visiting medical institution information, another is the medical examination and treatment information, and the other is post-medical treatment information.

The MIR may be generated by including a process of inputting and storing the pre-visiting medical institution information through the terminal of the membershiped information provider; a process of inputting and storing the medical examination and treatment information of the user after treating the user at a medical institution; and a process of inputting and storing the post-medical treatment information for the medical treatment through the terminal of the membershiped information provider.

After the MIR is generated, a process of storing the MIR in a search DB so that the MIR is searched may be further included.

The MIR stored in the search DB may be searchable on the terminal of the membershiped information provider and/or another user terminal.

The MIR stored in the search DB may be searchable on a medical institution terminal of the medical institution and/or a medical institution terminal of another medical institution.

The search may be performed on at least one information selected from the group consisting of pre-visiting medical institution information, the medical examination and treatment information, and post-medical treatment information.

The pre-visiting medical institution information includes a health questionnaire.

The health questionnaire may be provided by the terminal of the membershiped information provider or the MIR server.

The medical examination and treatment information may be input through the terminal of the membershiped information provider.

The medical examination and treatment information may be further input through the medical institution terminal of the medical institution.

The medical examination and treatment information may include a disease name for the user's disease and may further include therapeutic drug information and/or treatment method information.

After the process of receiving and storing the medical examination and treatment information, a process of receiving and storing a medical institution information that has treated the user.

The medical institution information may be input through the terminal of the membershiped information provider or the medical institution terminal of the medical institution.

After the process of inputting and storing the pre-visiting medical institution information, a process of selecting a medical category corresponding to the input pre-visiting medical institution information may be further included.

The medical category is a plurality of items in which the plurality of items are provided by sub-classifying one or more selected from the group consisting of a medical department, a type of symptom, a site of symptom, and a type of disease, and wherein the medical category is selected from at least one of the plurality of items.

After the process of selecting the medical category, a process of providing a list of medical institutions corresponding to the selected medical category to the terminal of the membershiped information provider and making an appointment with any one medical institution that is selected from the provided list of medical institutions by the terminal of the membershiped information provider.

The medical examination and treatment information may further include medical treatment information of the medical institution regarding the medical treatment.

The post-medical treatment information is further input through the medical institution terminal of the medical institution.

The MIR may further include a personal health record (PHR) stored in a government or public institution.

The medical treatment may include at least one of the user's on-site medical treatment and/or the user's remote medical treatment.

When the category of interest is healthcare, the MIR may be a healthcare record of an user selected from the membershiped information provider and a patients managed by the membershiped information provider; and the MIR include: (i) a MIR edition including, as pre-visiting medical institution information, information on symptoms of a disease, disorder, or condition; and (ii) a MIR edition including, as post-medical treatment information, information on the user's progress after treatment.

The pre-visiting medical institution information is generated by inputting information on the symptoms of the disease, disorder, or condition twice or more at time intervals, and the post-medical treatment information may be generated by inputting information on the progress after the treatment twice or more at time intervals.

The terminal environment-controlling instruction may be selectively provided in response to the received category of interest.

The terminal environment-controlling instruction may be selectively provided in response to information on the topic of interest input at the time intervals.

The terminal environment-controlling instruction may be provided in response to the received category of interest and the MIR edition.

The information editing module is a control command and/or an application utilize a text input module, an image generating module (or a drawing module), a touch input module, a camera, a microphone, a location recognition module (or a GPS module), and/or a sensor module for recording information in the terminal of the membershiped information provider.

The information sharing setting module may be received information to determine i) a range of a membershiped user in the MIR server to who the MIR is accessible; and/or ii) the MIR to be accessible to the membershiped user in the MIR server and/or a range of information in the MIR to be accessible to the membershiped user.

An edition may be assigned to at least two or more of the plurality of time-series information received in the time-series manner to generate a plurality of MIR editions, the plurality of MIR editions each include meta information, and the plurality of MIR editions may be incorporated in the MIR.

The MIR server may select the information record sheet depending on the information on the topic of interest input at the time intervals or the edition assigned to the MIR edition and then provide the selected information record sheet to the terminal of the membershiped information provider.

The MIR server may (i) selectively provide at least one of a plurality of information record sheets and provide the selected at least one information record sheet to the terminal of the membershiped information provider so that the membershiped information provider may selectively input standardized information corresponding to the information on the topic of interest input at the time intervals or may (ii) select the at least one of the plurality of information record sheets and provide the selected at least one of information record sheet to the terminal of the membershiped information provider so that the membershiped information provider may selectively input standardized information corresponding to the edition.

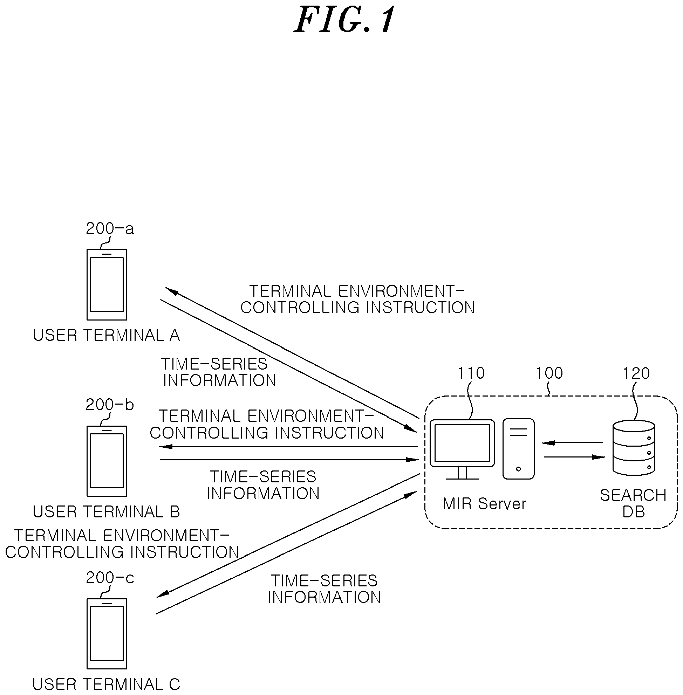

FIG. 1 is a configuration diagram illustrating an individual-directed information record sheet generating system according to the present disclosure. As shown in FIG. 1, a MIR server 110 is connected to a terminal 200-a (hereinafter, referred to as a `user terminal') of a membershiped information provider and terminals 200-b and 200-c (referred to as 'other users' terminals') of other membershiped information providers through a communication network, and a MIR application is installed in the user terminals 200-a, 200-b, and 200-c.

The MIR application of the user terminals 200-a, 200-b, and 200-c receives a terminal environment-controlling instruction provided by the MIR server 110. The MIR application may output an information record sheet on a screen of the terminal according to the received terminal environment-controlling instruction and receive information on the field of interest from the membershiped information provider.

Information on the field of interest input into the information record sheet by the membershiped information provider is transmitted to the MIR server 110 through the user terminal (the terminal of the membershiped information provider), and the MIR server 110 creates MIR using a plurality of pieces of information on the field of interest sequentially transmitted from each membershiped information provider.

The MIR server 110 may exist as a single server and, if necessary, may include a plurality of MIR servers.

The MIR application may be installed and executed on the user terminals 200-a, 200-b, and 200-c, but, if necessary, may be installed and executed on a terminal, such as a PC or tablet connected to a communication network. In addition, the MIR server 110 may provide terminal environment-controlling instructions in a web manner to a terminal in which the MIR application is not installed.

In an implementation of the present disclosure, a plurality of membershiped information providers respectively using the user terminals 200-a, 200-b, and 200-c are registered as members or associate members in the MIR server 110 or the MIR application.

In an implementation of the present disclosure, the terminal environment-controlling instruction may be provided from the MIR server 110 when a membershiped information provider inputs information on a field of interest using the MIR application.

In an implementation of the present disclosure, the terminal environment-controlling instruction may be included in the MIR application installed on the terminal of the membershiped information provider. If the MIR application installed on the terminal of the membershiped information provider includes a terminal environment-controlling instruction, the terminal environment-controlling instruction is not provided from the MIR server 110.

A tracking module (not shown) included in the MIR server 110 of the present disclosure may provide a development history of the MIR upon a request of the user terminals 200-a, 200-b, and 200-c owned by the membershiped information provider and/or member user. The development history of MIR includes the history of creation and change of information received for creation of the MIR.

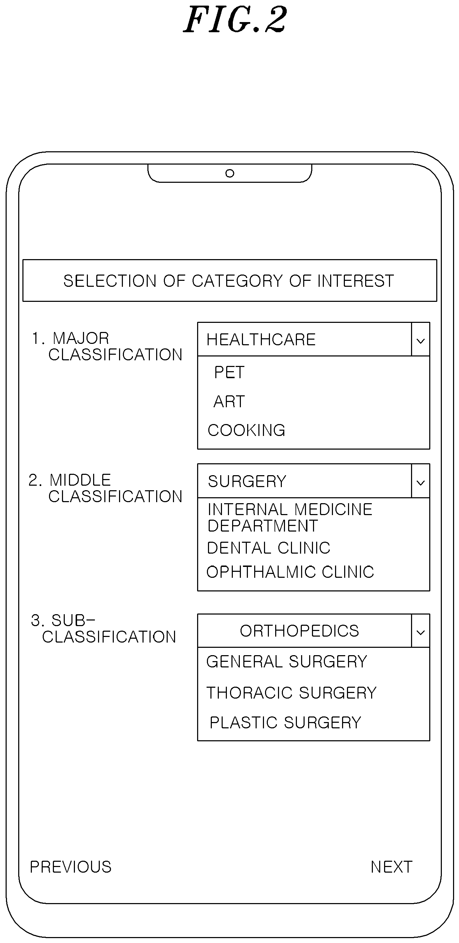

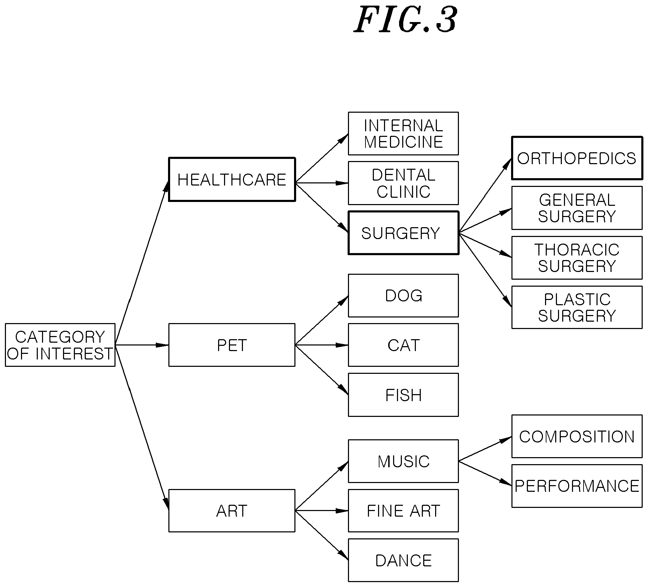

FIG. 2 is an exemplary diagram illustrating an embodiment of a screen configuration for selecting a category of interest in a MIR application according to an implementation of the present disclosure. FIG. 3 is an exemplary diagram illustrating an embodiment of a category of interest including hierarchical information levels according to an implementation of the present disclosure.

As shown in FIG. 2, the membershiped information provider may select a category of interest to input information on the field of interest.

In an implementation of the present disclosure, a category of interest may include a plurality of hierarchical information levels. FIG. 3 shows an embodiment of a category of interest including a plurality of hierarchical information levels. A category of interest including a plurality of hierarchical information levels may include a plurality of categories for each layer.

In another implementation of the present disclosure, a category of interest may include a single information level. The category of interest including a single information level may include a plurality of categories.

The MIR server 110 may provide the category of interest to the terminal of the membershiped information provider 200 in order to receive information on the topic of interest from the membershiped information provider.

In an implementation of the present disclosure, the category of interest may be provided in real time from the MIR server 110.

In another implementation of the present disclosure, the category of interest may be included in the MIR application.

The membershiped information provider may select one of the categories of interest executed in or output from the MIR application and provides the selected category of interest to the MIR server 110.

In an implementation of the present disclosure, when selecting any one category among categories of interest including the hierarchical information levels, the membershiped information provider may select the category of interest at the lowest level.

In another implementation of the present disclosure, when the MIR server 110 includes an information record sheet corresponding to a level prior to the lowest level, the membershiped information provider may select a category of interest at the level prior to the lowest level.

In another implementation of the present disclosure, the membershiped information provider may select one of the categories of interest including a single information level.

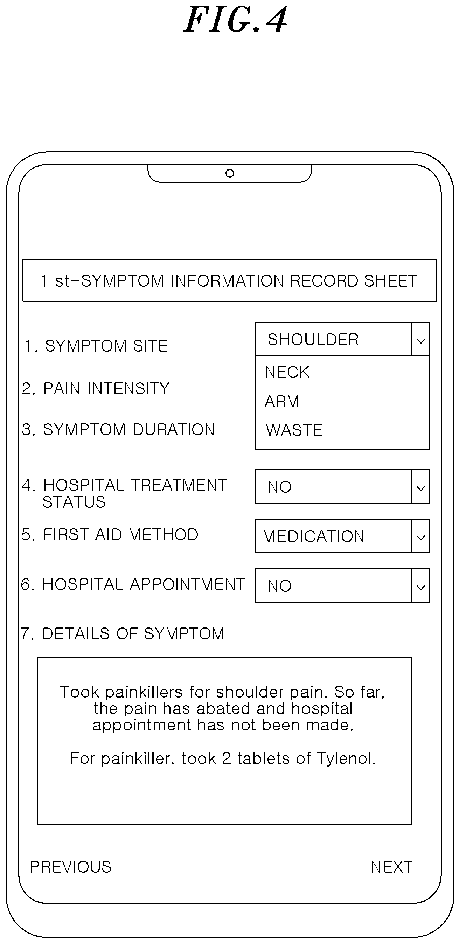

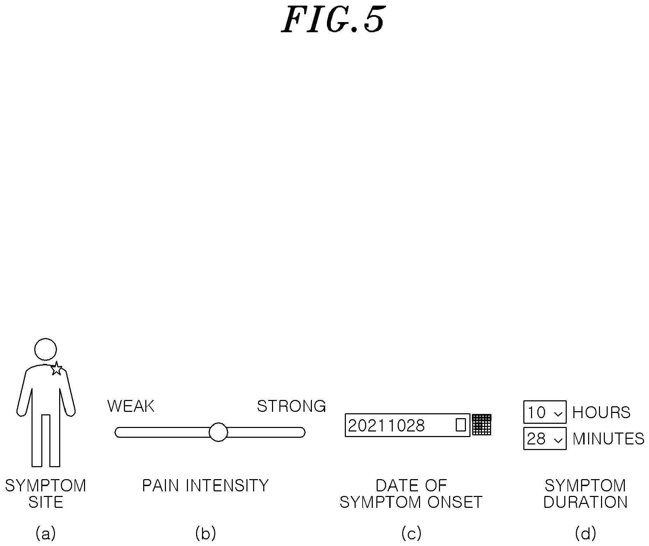

FIG. 4 is an exemplary diagram illustrating an embodiment of a screen configuration for outputting an information record sheet in a MIR application according to an implementation of the present disclosure. FIG. 5 is an exemplary diagram illustrating an embodiment of an information editing module that may be included in an information record sheet according to an implementation of the present disclosure.

As shown in FIGS. 4 and 5, an information record sheet output to the terminal of the membershiped information provider may receive information on a topic of interest by the membershiped information provider.

In an implementation of the present disclosure, the information provision sheet may be provided to be different for each category of interest selected by the membershiped information provider, as in the implementation of FIG. 3.

In another implementation of the present disclosure, the information provision sheet may be provided to be different for each edition (hereinafter referred to as 'edition'), which is a step for inputting information on the topic of interest.

In another implementation of the present disclosure, informational sheets may be provided differently for each category of interest and edition.

The information provision sheet includes an information editing module. The information editing module may include a control instruction and/or application for using a text input module, an image generating module (or a drawing module), a touch input module (or a touch module), a camera, a microphone, and a location recognition module (or a GPS module), and or a sensor module for recording information in the terminal of the membershiped information provider.

As shown in FIG. 5, the information editing module may include various graphic user interfaces (GUIs) included in the information record sheet.

In an implementation of the present disclosure, the GUI of the information editing module may include a GUI through coordinate or image recognition, as shown in (a) of FIG. 5. For example, (i) the GUI of the information editing module may recognize a location selected by the membershiped information provider through touch input in an image of a human body shape displayed on the screen, and (ii) the GUI of the information editing module may recognize a location selected by the membershiped information provider through touch input in a map-shaped image displayed on the screen.

At this time, the image output to the terminal of the membershiped information provider may include one or more of 2D or 3D images.

In another implementation of the present disclosure, the GUI of the information editing module may include a scroll bar, etc., as shown in (b) of FIG. 5. For example, when inputting the intensity of pain, concentration of liquid, etc., since they cannot be input accurately in a text form, they may be input in a numerical form using a scroll bar, etc.

In another implementation of the present disclosure, the GUI of the information editing module may include a calendar as shown in (c) of FIG. 5. For example, when inputting date information, if the date information is input through text, membershiped information providers may input only numbers or a combination of numbers and the year, month, and day, so it may be difficult to use as standardized data. Therefore, by providing a GUI, such as a calendar, an embodiment of selecting and inputting a date from a displayed calendar may be performed.

In another implementation of the present disclosure, the GUI of the information editing module may include a combo box for selecting a time, as shown in (d) of FIG. 5. For example, when inputting time information, as with date information, different types of text input may occur depending on the membershiped information provider, so a GUI, such as a combo box for selectively inputting time information for use as standardized data may be used.

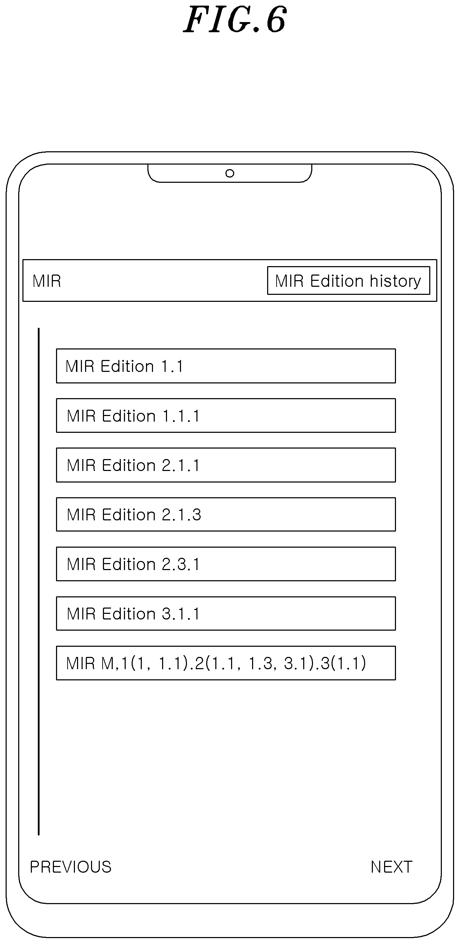

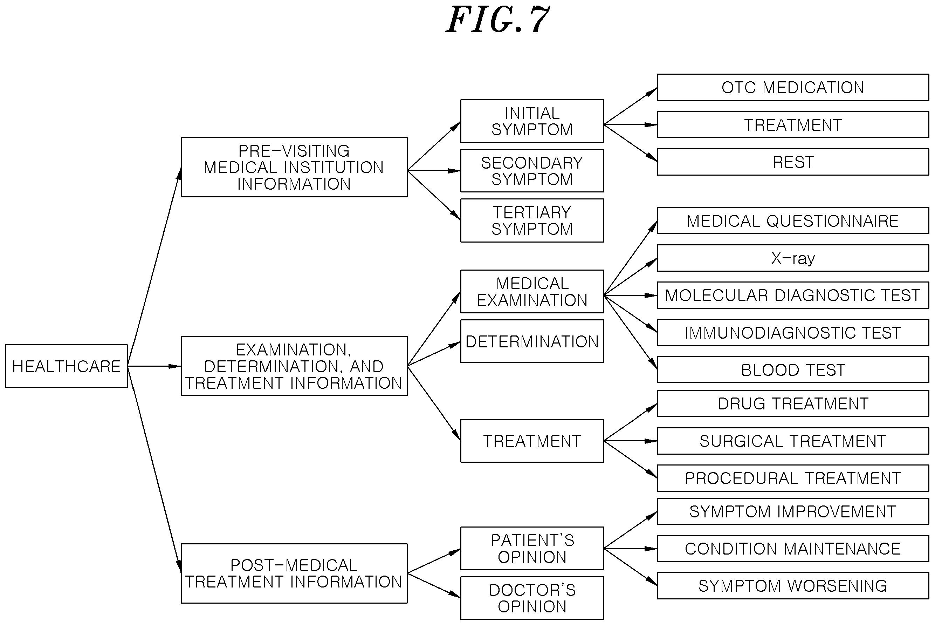

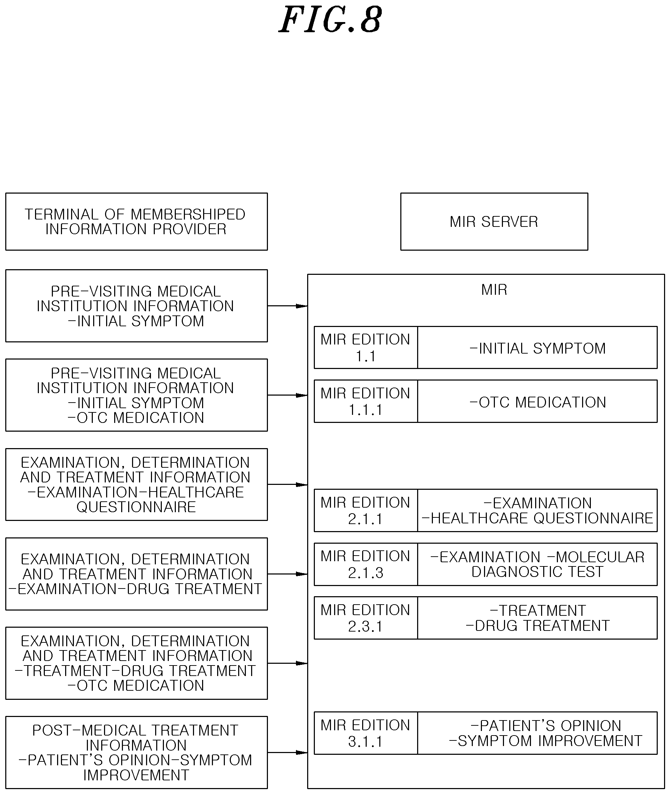

FIG. 6 is an exemplary diagram illustrating an embodiment of a screen configuration for outputting an index of a MIR edition generated using a MIR application according to an implementation of the present disclosure. FIG. 7 is an exemplary diagram illustrating an embodiment in which a hierarchically configured edition is provided by the MIR server according to an implementation of the present disclosure. FIG. 8 is an exemplary diagram illustrating an embodiment in which an edition index is generated by the MIR server allocating an edition to time-series information provided from a user terminal according to an implementation of the present disclosure.

FIG. 6 shows an embodiment in which when a MIR edition is generated, a corresponding edition index is output to the user terminal of the membershiped information provider. In other words, the membershiped information provider inputted information on topics of interest six times sequentially using the information provision sheet. In response to this, the MIR server received information on the topic of interest six times, which is chronological information, and generated a MIR edition to which a total of 6 editions were assigned. In response, as shown in the example of FIG. 6, the MIR server may provide an edition index through which the generated MIR edition may be read to the terminal of the membershiped information provider.

In an implementation of the present disclosure, the MIR server automatically allocates an edition in response to information on the transmitted topic of interest.

In another implementation of the present disclosure, the MIR server may generate and allocate a separate edition according to a request based on the needs of the membershiped information provider.

In another implementation of the present disclosure, the MIR server may automatically allocate an edition in response to information on the transmitted topic of interest and may additionally generate and allocate a separate edition upon request based on the needs by the membershiped information provider.

Accordingly, the indexes of a total of six generated MIR editions are displayed on the screen of the terminal of the membershiped information provider. In addition, when a MIR is generated through six MIR editions, an index for the MIR including information on the entire input topics of interest may also be output.

In addition, on the displayed screen, a `MIR edition history' button may be generated to check a creation procedure of each generated MIR edition while receiving information on each topic of interest sequentially.

The MIR server may automatically generate MIR edition history using information on the input topic of interest and information on the MIR edition generated to correspond thereto.

In an implementation of the present disclosure, the MIR edition history may include information on date and time information at which each MIR edition was generated.

In another implementation of the present disclosure, MIR edition history may include modified history information when the MIR edition generated using information on the topic of interest input by the membershiped information provider is modified. Modified history information includes contents before and after content modification by the membershiped information provider. Accordingly, it is possible to check which part of the contents of the MIR edition has been changed.

In another implementation of the present disclosure, MIR edition history may include disclosure and sharing history information of MIR editions. For example, the membershiped information provider may set the disclosure and sharing of the already generated MIR edition after or before the creation of the MIR is completed. At this time, information, such as an index of at least one MIR edition selected from the group consisting of a period of disclosure and sharing, a target person, the disclosure, and a disclosure target, is generated as history information. The MIR server may include the generated disclosure and sharing history information in the MIR edition history.

The MIR server may provide MIR edition history so that it may be output through graphic visualization.

In an implementation of the present disclosure, the MIR edition history may express information in a graph format.

In another implementation of the present disclosure, the MIR edition history may express information in the form of a tree structure.

In another implementation of the present disclosure, the MIR edition history may express information in the form of a 3D image.

Editions may be allocated to correspond to a selected category of interest. In addition, as shown in FIG. 7, editions have a hierarchical structure. Each edition is classified in the same layer and may be extended to one or more sub-layers.

In addition, when the membershiped information provider uses a related information sheet when information on a topic of interest is provided sequentially, an edition corresponding to the information sheet may be allocated.

In an implementation of the present disclosure, when a disease occurs in the membershiped information provider, the assigned edition index may be allocated as follows.
1) Pre-visiting medical institution information, 2) examination, determination, and treatment information following a visit to a medical institution, and 3) post-medical treatment information may be recorded. For example:
   1) Pre-visiting medical institution information may be classified into 1.1) initial symptoms, 1.2) secondary symptoms, 1.3) tertiary symptoms, etc. as the disease occurs. In addition, sub-editions classified from 1.1) initial symptoms may be extended to 1.1.1) Overthe-counter (OTC) drug medication, 1.1.2) treatment, 1.1.3) rest, etc.
   2) Medical institution visit information, including examination, determination, and treatment information, may be classified into 2.1) examination, 2.2) determination, 2.3) treatment, etc. In addition, the sub-editions classified from 2.1) examination may be extended to 2.1.1) doctor's questionnaire, 2.1.2) X-ray, 2.1.3) molecular diagnostic test, 2.1.4) immunodiagnostic test, 2.1.5) blood test, etc. In addition, sub-editions classified from 2.3) treatment may be extended to 2.3.1) drug treatment, 2.3.2) surgical treatment, 2.3.3) procedural treatment, etc.
   3) Post-medical treatment information may be classified into 3.1) user opinion, 3.2) doctor opinion, etc. In addition, 3.1) sub-editions classified from user opinion may be extended to 3.1.1) symptom improvement, 3.1.2) condition maintenance, 3.1.3) symptom worsening, etc.

When the membershiped information provider records and provides information on the topic of interest on the information record sheet according to the contents of the information on the topic of interest to be recorded, the MIR server may automatically allocate an index of the edition according to the contents and generates the information on topics of interest as a MIR edition.

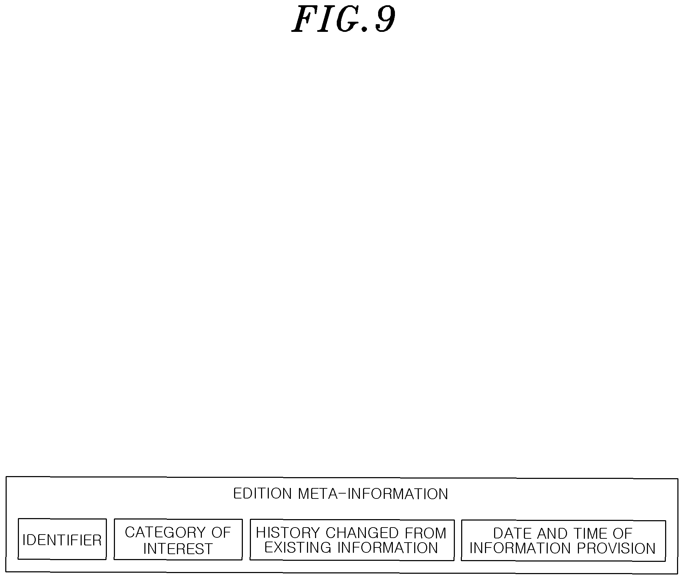

FIG. 9 is an exemplary diagram illustrating an embodiment of meta information included in an edition allocated by a MIR server according to an implementation of the present disclosure. As shown in FIG. 9, in the terminal of the membershiped information provider, the membershiped information provider generates information on topics of interest sequentially using an information record sheet and transmits it to the MIR server.

Referring to FIGS. 7 to 9, generated information on topics of interest is as follows.
1.1) Pre-visiting medical institution information - first symptom
1.1.1) Pre-visiting medical institution information - first symptom - OTC medication
2.1.1) Examination, determination and treatment information - examination - doctor's questionnaire
2.1.3) Examination, diagnosis and treatment information - examination - molecular diagnostic test
2.3.1) Examination, judgment and treatment information - treatment - drug treatment
3.1.1) Post-medical treatment information - user opinion - symptom improvement

Information on each input topic of interest is transmitted to the MIR server sequentially in the order in which it is written. The MIR server generates a MIR edition by allocating an edition corresponding to the information on topics of interest transmitted sequentially.

After generating a final MIR edition, the MIR server may generate an MIR including a MIR edition of each of a plurality of MIR editions 1.1), 1.1.1), 2.1.1), 2.1.3), 2.3.1), and 3.1.1).

The MIR server may generate `edition meta-information' in the edition, include it in the MIR edition, and store the same. Edition meta information may include an identifier of an edition, category of interest information, details of changes from existing information, date and time of information provision, location information corresponding to information on the topic of interest, etc.

In an implementation of the present disclosure, one or more of the information included in the edition meta information may be extracted from information on a topic of interest transmitted from the terminal of the membershiped information provider to be generated. For example, it may include the date and time of input of information on the topic of interest. In addition, it may include category of interest information.

In another implementation of the present disclosure, one or more of the information included in the edition meta information may be generated upon being extracted from information generated while receiving information on the topic of interest from the MIR server. For example, it may include the date and time of reception. In addition, it may include category of interest information.

In another implementation of the present disclosure, information included in edition meta information may be generated including the assigned edition. For example, an edition may be generated to correspond to information on a topic of interest, and the corresponding edition may be included.

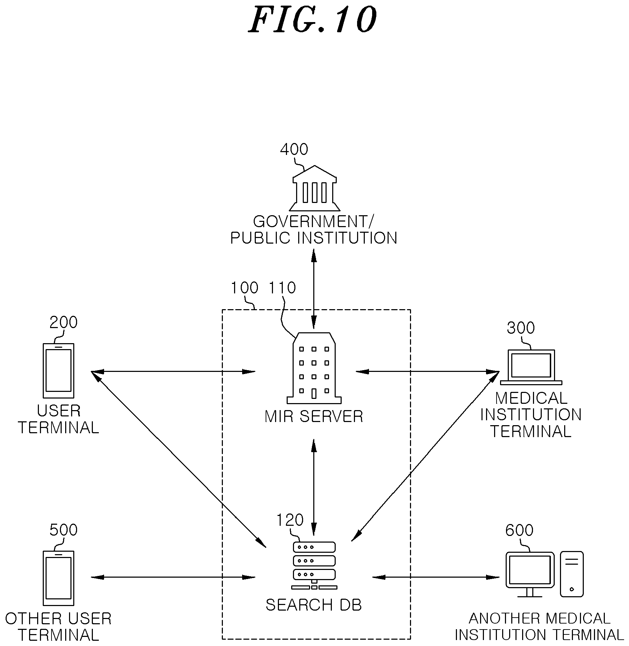

FIG. 10 is a configuration diagram illustrating an individual-directed information record sheet generating system according to another implementation of the present disclosure. As shown in FIG. 10, an individual-directed information record sheet generating system 100 includes an individual-directed information record sheet generating server 110 (hereinafter referred to as a `MIR server') and a search DB 120.

The MIR server 110 may be connected to a plurality of user terminals through a communication network and may receive information input to each user terminal. In addition, the MIR server 110 may provide each information requested by a plurality of user terminals.

The MIR server 110 may be connected to one or more cloud servers (not shown) through a communication network, so that user terminals may request and receive healthcare records stored in the cloud server. User terminals may store healthcare records that may generate an individual-directed information record sheet from the user terminal to a cloud server used by an individual. In this case, the MIR server 110 may receive the healthcare records stored in the cloud server according to approval of the user terminal.

The MIR server 110 may be connected to a plurality of medical institution terminals through a communication network and may receive information input from each medical institution terminal. In addition, the MIR server 110 may provide each information requested by a plurality of medical institution terminals.

The MIR server 110 may forecast a user's disease through analysis of pre-visiting medical institution information provided from the user terminal 200. The MIR server 110 may select a disease and a medical subject for the disease in a medical category in response to the forecast user's disease.

When the selected disease and medical subject are selected, the MIR server 110 generates a list of corresponding medical institutions. The generated list of medical institutions includes medical institutions that may treat the user.

The list of medical institutions may include a medical institution registered as a member in the individual-directed information record sheet generating system 100. The list of medical institutions may include a medical institution located close to the user's residence or a real-time current location (measured through the user terminal).

The list of medical institutions may include medical institutions that previously treated the user.

The list of medical institutions may include a medical institution ranked higher among medical institutions recommended by other users.

In an embodiment of the present disclosure, a recommendation of a medical institution may use a ranking calculated by giving weight to `post-medical treatment information' among a plurality of individual-directed information record sheets stored in the search DB.

In another embodiment of the present disclosure, the recommendation of a medical institution may use a ranking calculated according to medical institution evaluation items included in `post-medical treatment information' among a plurality of individual-directed information record sheets stored in the search DB.

The MIR server 110 may request a reservation at a medical institution by providing the generated list of medical institutions to the user terminal 200.

The MIR server 110 may make a medical treatment appointment requesting medical treatment for the user at the selected medical institution 300 in response to an input from a user terminal that selects one medical institution from the list of medical institutions provided.

The MIR server 110 may omit the medical treatment appointment in response to the user terminal 200 not selecting a medical institution.

The MIR server 110 may be connected to a government/public institution terminal 400 through a communication network and may request personal health records (PHR) information that may be included in the individual-directed information record sheet. In addition, the MIR server 110 may receive PHR information provided from the information/public institution terminal 400.

The MIR server 110 may be connected to the search DB 120 through a communication network and may store the generated individual-directed information record sheet in the search DB .

In an implementation of the present disclosure, the MIR server 110 may be configured separately from the search DB 120, and the MIR server 110 and the search DB 120 may be operated independently.

In another implementation of the present disclosure, the MIR server 110 may be configured to include a search DB 120, and the MIR server 110 and the search DB 120 may be operated together.

The search DB 120 may receive an individual-directed information record sheet generated by the MIR server 110 and provide an individual-directed information record sheet in response to a search request from an external source.

The search DB 120 may store a plurality of individual-directed information record sheets each generated by the MIR server 110 using information input from a plurality of user terminals and provide an individual-directed information record sheet corresponding to search requested by the user terminals and/or medical institution terminals.

The search DB 12 may provide an individual-directed information record sheet corresponding to searches requested by user terminals and/or medical institution terminals as a paid service. The user terminals and/or medical institution terminals may pay a certain fee to receive an individual-directed information record sheet stored in the search DB.

For example, the individual-directed information record sheet stored in the search DB 120 may be provided to a member who pays a predetermined fee in advance. The user terminals 200 and 500 and/or medical institution terminals 300 and 600 may be provided with the individual-directed information record sheet when payment of the fee corresponding to the individual-directed information record sheet searched in the search DB 120 is completed.

In addition, the user terminal 200 and/or the medical institution terminal 300 that has provided information to generate the individual-directed information record sheet in the MIR server 110 may search the individual-directed information record sheet without paying a fee.

The government/public institution terminal 400 included in the government and/or public institution may provide the user's PHR information requested by the MIR server 110.

In an implementation of the present disclosure, the user's PHR information may be requested by the MIR server 110 from the government/public institution terminal 400, and the MIR server 110 may receive the information.

In another implementation of the present disclosure, the user's PHR information may be requested by the user terminal 200 from the government/public institution terminal 400, and the MIR server 110 may receive the information.

In another implementation of the present disclosure, the user's PHR information may be requested by the user terminal 200 from the information/public institution terminal 400, and the user terminal 200 may receive the information. Thereafter, the user terminal 200 may provide the received PHR information to the MIR server 110.

The user's PHR information provided by the government/public institution 400 is one or more of a plurality of information stored to correspond to the user. For example, PHR information may include information stored in government and/or public institutions, such as height, weight, vision, blood type, vaccinations, genetic diseases, allergies, infectious disease records, health insurance, healthcare records from medical institutions, drug prescription records, medical images, blood pressure, blood sugar, heart rate, etc.

In an embodiment of the present disclosure, the PHR information stored in the government/public institution terminal 400 may be a health record directly recorded by the user.

In another embodiment of the present disclosure, the PHR information stored in the government/public institution terminal 400 may be a health record provided by a medical institution.

In another embodiment of the present disclosure, the PHR information stored in the government/public institution terminal 400 may be a health record provided by the government/public institution.

In another embodiment of the present disclosure, the PHR information stored in the government/public institution terminal 400 may be at least one health record among those selected from the group consisting of health records directly recorded by the user, health records provided by medical institutions, and health records provided by government/public institutions.

The user terminal 200 is a terminal of a membershiped information provider that uses the individual-directed information record sheet generating system 100. The user terminal 200 may be installed with an application that allows input of pre-visiting medical institution information, medical examination and treatment information, and post-medical treatment information. The application may be connected to the MIR server 110 through a communication network and provide input information. The application installed on the user terminal 200 is a MIR application.

The user terminal 200 may be a terminal registered as a member to use the individual-directed information record sheet generating system 100. Accordingly, the user terminal 200 may provide the MIR server 110 with information that may generate an individual-directed information record sheet.

In an implementation of the present disclosure, the user terminal 200 may be connected to the MIR server 110 through a communication network using a web application.

In another implementation of the present disclosure, the user terminal 200 may be connected to the MIR server 110 through a communication network using an installed dedicated application.

The user terminal 200 may input healthcare records using the above application and provide them to the MIR server 110.

When a symptom occurs, the user may input healthcare records, such as pre-visiting medical institution information, into the user terminal 200 to generate a healthcare record sheet for the corresponding symptom and provide the same to the MIR server 110.

In an implementation of the present disclosure, the healthcare record input by the user terminal 200 includes pre-visiting medical institution information, medical examination and treatment information, and post-medical treatment information.

In another implementation of the present disclosure, the healthcare record input by the user terminal 200 includes pre-visiting medical institution information, medical examination and treatment information, and post-medical treatment information, and may further include medical institution information if necessary.

The user terminal 200 may input the pre-visiting medical institution information using an application. In an implementation of the present disclosure, a health questionnaire included in pre-visiting medical institution information may be included in the application. In another implementation of the present disclosure, a health questionnaire included in pre-visiting medical institution information may be provided from the MIR server 110 and output using an application.

The user terminal 200 may provide the medical examination and treatment information to the MIR server 110 using an application. In an implementation of the present disclosure, the user may input medical examination and treatment information generated according to treatment at a medical institution into the user terminal 200 and provide the same to the MIR server 110. In another implementation of the present disclosure, the user may provide medical examination and treatment information provided from the medical institution terminal 300 according to the medical treatment of the medical institution to the MIR server 110.

The user terminal 200 may provide medical institution information to the MIR server 110 using an application. In an implementation of the present disclosure, the user may input information on the medical institution that performed the treatment into the user terminal 200 and provide the information to the MIR server 110. In another implementation of the present disclosure, the user may provide medical institution information provided from the medical institution terminal of the medical institution that performed the treatment to the MIR server 110.

The user terminal 200 may provide post-medical treatment information to the MIR server 110 using an application. In an implementation of the present disclosure, the user may input the post-medical treatment information to the user terminal 200 according to treatment at a medical institution and provide the information to the MIR server 110. In another implementation of the present disclosure, the user may provide post-medical treatment information provided from the medical institution terminal 300 according to the treatment at the medical institution to the MIR server 110.

The user terminal 200 may connect to the MIR server 110 and check the healthcare records provided by the user using the application. The user terminal 200 may check the medical institution information, including previously provided pre-visiting medical institution information, medical examination and treatment information, and post-medical treatment information, on the MIR server 110. The user terminal 200 may modify pre-stored healthcare records as needed.

The user terminal 200 may search the search DB 120 for necessary healthcare records using the application. In an implementation of the present disclosure, the user terminal 200 may search the user's healthcare records. In another implementation of the present disclosure, the user terminal 200 may search another user's healthcare records.

The medical institution terminal 300 is a terminal included in a medical institution that treats users. The medical institution terminal 300 is connected to the MIR server 110 through a communication network and may transmit/receive information.

The medical institution terminal 300 may be a terminal that has registered as a member to use the individual-directed information record sheet generating system 100. Accordingly, the medical institution terminal 300 may provide the MIR server 110 with information that may be used to generate an individual-directed information record sheet of the user.

In an implementation of the present disclosure, the medical institution terminal 300 may be connected to the MIR server 110 through a communication network using a web application.

In another implementation of the present disclosure, the medical institution terminal 300 may be connected to the MIR server 110 through a communication network using an installed dedicated application.

The medical institution terminal 300 may input the user's healthcare record using the above application and provide the same to the MIR server 110.

In an implementation of the present disclosure, the medical institution terminal 300 may directly provide the user's healthcare record to the MIR server 110.

In another implementation of the present disclosure, the medical institution terminal 300 may provide the user's healthcare record to the user terminal 200 and the user terminal 200 may provide the received the healthcare record to the MIR server 110.

When a user's medical treatment is requested, the medical institution may input medical information to generate the user's healthcare record sheet and provide the same to the MIR server 110.

In an implementation of the present disclosure, the healthcare record input by the medical institution terminal 300 may include the user's medical examination and treatment information and/or medical institution information.

In another implementation of the present disclosure, the healthcare record input by the medical institution terminal 300 may include the user's medical examination and treatment information and/or medical institution information and may further include post-medical treatment information, if necessary.

When the user provides the MIR server 110 with information requesting a medical treatment appointment at the corresponding medical institution, the medical institution terminal 300 may receive the user's medical treatment appointment information from the MIR server 110.

In an implementation of the present disclosure, when the medical institution terminal 300 is registered as a member of the individual-directed information record sheet generating system 100, the medical institution terminal 300 may receive the user's medical treatment appointment information. The medical institution terminal 300 may receive the user's medical treatment appointment information and provide appointment completion information to the user terminal 200.

The medical institution terminal 300 may store healthcare records generated according to the user's medical treatment.

In an implementation of the present disclosure, the medical institution terminal 300 may input and store healthcare records generated by a medical professional according to the user's treatment by using an application.

In another implementation of the present disclosure, the healthcare records input by a medical professional according to the user's treatment are stored in an electronic healthcare record (EMR), and the medical institution terminal 300 may refer to the healthcare records stored in the EMR using a dedicated application.

The government/public institution terminal 400 may provide the user's PHR information to the MIR server 110.

In an implementation of the present disclosure, one or more government/public institution terminals 400 may be provided in the government and each public institution and may be a plurality of terminals that may provide each user's PHR information to the MIR server 110 connected through a communication network.

In another implementation of the present disclosure, the government/public institution terminal 400 may be a single terminal that receives the user's PHR information from other terminals respectively held by the government and each public institution and provides the same to the MIR server 110 connected through a communication network.

In another implementation of the present disclosure, the government/public institution terminal 400 may be a terminal that stores and manages each user's PHR information held by the government and each public institution.

Another user terminal 500 may access the search DB 120, in which a plurality of individual-directed information record sheets generated by the MIR server 110 are stored, and search for the healthcare records.

In an implementation of the present disclosure, the other user terminal 500 is a user terminal that has registered as a member, and the search DB 120 may search for and provide a healthcare record corresponding to a search word input from the other user terminal 500 and may request payment of expenses if necessary.

A search word input from the other user terminal 500 is searched from any one among pre-visiting medical institution information, medical examination and treatment information, medical institution information, and post-medical treatment information included in the healthcare records of the search DB 120. The search DB 120 may provide results corresponding to the search word to the other user terminals 500.

The other user terminal 500 may be another user (another membershiped information provider) who has written a healthcare record, or may be a general user who has not written a healthcare record. The other user terminal 500 is a terminal owned or managed by a plurality of users.

In the present disclosure, the user terminal 500, which is different from the user terminal 200, is preferably a mobile terminal owned by the user, but is not limited thereto and may include a terminal, such as a general PC.

The other medical institution terminal 600 may access the search DB 120, in which a plurality of individual-directed information record sheets generated by the MIR server 110 are stored, and search for the healthcare records.

In an implementation of the present disclosure, the other medical institution terminal 600 is a medical institution terminal that has registered as a member, and the search DB 120 may search for healthcare records corresponding to a search word input from the other medical institution terminal 600 and provide a searched healthcare record sheet to the other medical institution terminal 600.

In another implementation of the present disclosure, the other medical institution terminal 600 is a general medical institution terminal, and the search DB 120 may search and provide healthcare records corresponding to search of the other medical institution terminal 600 of the a medical institution, but may request payment of costs if necessary.

A search word input from the other medical institution terminal 600 is searched on any one of pre-visiting medical institution information, medical examination and treatment information, medical institution information, and post-medical treatment information included in the healthcare records of the search DB 120. The search DB 120 may provide results corresponding to the search word to the other medical institution terminal 600.

The other medical institution terminal 600 may be a medical institution that participated in generating a healthcare record sheet or may be a general medical institution that has not participated in generating a healthcare record. The other medical institution terminal 600 is a terminal owned by each of a plurality of medical institutions.

In the present disclosure, the medical institution terminal 300 and the other medical institution terminal 600 may include a mobile terminal and/or a general PC owned by a medical institution.

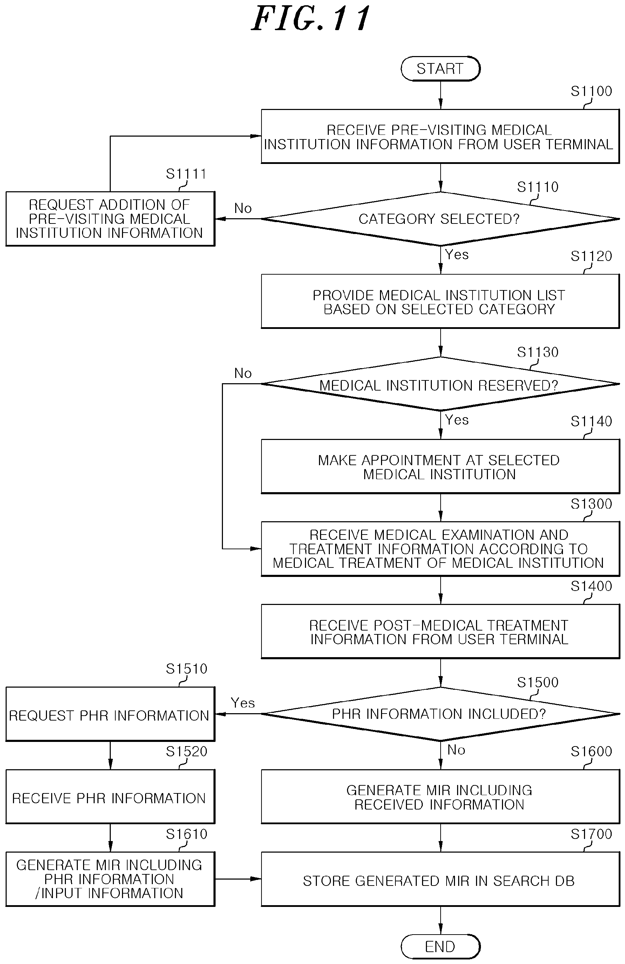

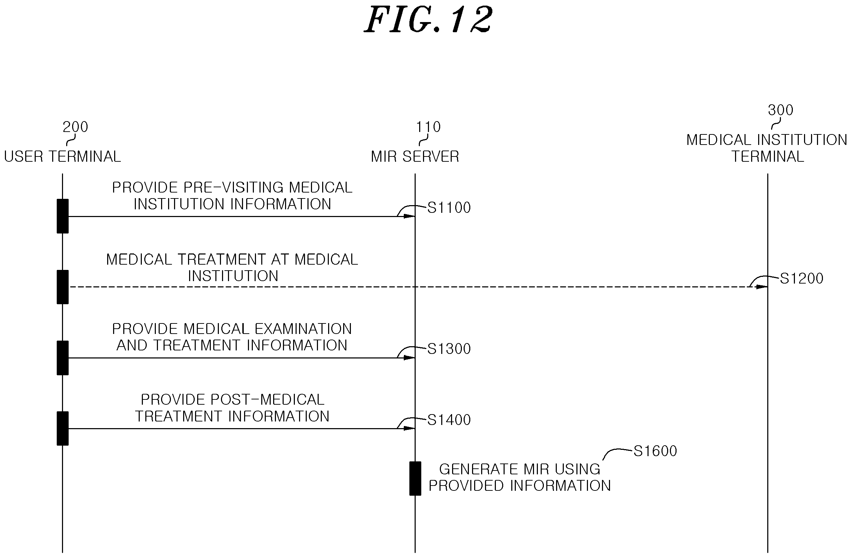

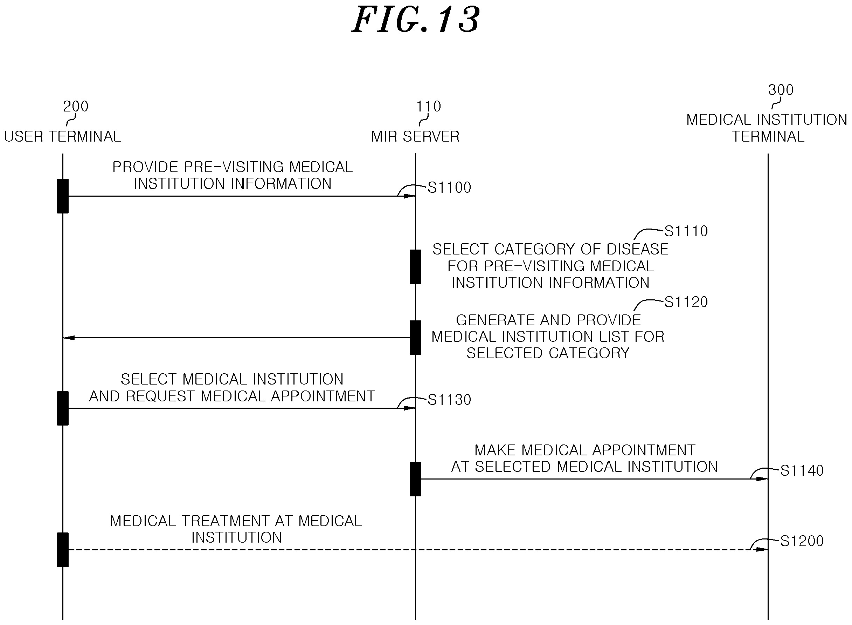

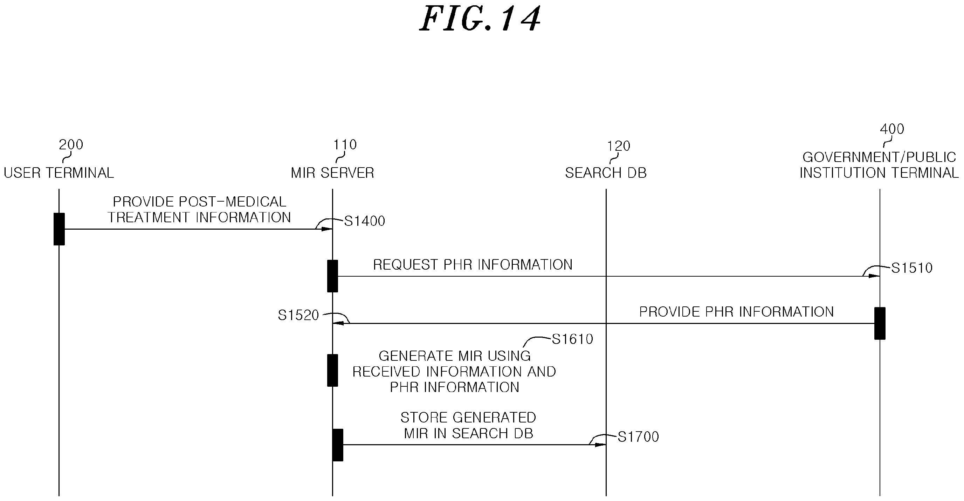

FIG. 11 is a flowchart illustrating a method of generating an individual-directed information record sheet according to another implementation of the present disclosure. FIG. 12 is a flowchart illustrating generating an individual-directed information record sheet according to another implementation of the present disclosure. FIG. 13 is a flowchart for making an appointment for a medical institution in an individual-directed information record sheet generating system according to another implementation of the present disclosure. FIG. 14 is a flowchart for including PHR information in an individual-directed information record sheet generating system according to another implementation of the present disclosure.

As shown in FIGS. 11 to 14, the method of generating an individual-directed information record sheet is as follows.

A user having a symptom of a disease may input pre-visiting medical institution information using a dedicated application or web application installed on the user terminal 200 and provide the information to the MIR server 110 of the individual-directed information record sheet generating system 100 (S 1100).

The pre-visiting medical institution information may be input in a text form, and files, such as audio, image, and video may be attached as needed. The pre-visiting medical institution information may include a health questionnaire provided by a dedicated application or the MIR server 110. The user inputs responses in response to the health questionnaire output from the user terminal 200 and provides the pre-visiting medical institution information, including the input responses, to the MIR server 110.

The MIR server 110 may select a medical category by analyzing the received pre-visiting medical institution information (S1110).

The medical category analyzes the symptom and/or contents of the health questionnaire recorded in the pre-visiting medical institution information and derives at least one selected from the group consisting of information, such as a position in which the user's symptom occurred, type of the symptom, period of the symptom occurrence, degree of pain of the symptom, range of the symptom, cause of the symptom occurrence, etc.

The MIR server 110 may select a medical category by analyzing the type of the symptom and the site of the symptom included in pre-visiting medical institution information.

In an implementation of the present disclosure, the medical category may be selected from one or more of a medical subject, type of symptom, site of symptom, or type of disease.

In another implementation of the present disclosure, the medical category may be an item classified from one or more of the medical subject, type of symptom, site of symptom, or type of disease, and any one or more medical categories among the plurality of items may be selected.

The medical subject, type of symptom, site of symptom, and type of disease included in the medical category may be expressed as follows.

For example:
i) The type of symptom may include muscle pain, neuralgia, abdominal pain, headache, toothache, menstrual pain, skin rash, burns, wounds, fractures, cough, etc.
ii) The site of symptom may include the head, face (eyes, ears, nose, and mouth), neck, chest, stomach, arms, wrists, hands, genitals, legs, ankles, and feet.
iii) The type of expected disease may include tonsillitis, pneumonia, tuberculosis, periodontitis, allergies, atopy, burns, hepatitis, gastritis, stomach ulcers, sexually transmitted diseases, arthritis, dry eyes, appendix, food poisoning, etc.
iv) The medical subject may include internal medicine, surgery, orthopedics, dermatology, obstetrics and gynecology, neurosurgery, dentistry, plastic surgery, pediatrics, and psychiatry.

The MIR server 110, which analyzes the pre-visiting medical institution information, may forecast the type of disease and select a medical subject corresponding to the forecast type of disease.

The MIR server 110 selects a medical category in which the disease is forecast based on the contents of the derived symptom. The medical category may include medical subjects for treating diseases.

For example, if the symptom is abdominal pain, a medical category, such as `internal medicine' may be selected.

For example, if the symptom is knee pain, medical categories, such as `orthopedics', `neurosurgery', or 'oriental medical clinic' may be selected.

As described above, one or more medical categories may be selected by analyzing the pre-visiting medical institution information.

If the MIR server 110 cannot select a medical category due to insufficient contents of the information received pre-visiting medical institution information, the MIR server 110 may request the user terminal 200 to add pre-visiting medical institution information (S1111). In response to the request for additional pre-visiting medical institution information, the user terminal 200 may re-perform step S1100 of receiving additional pre-visiting medical institution information from the user and providing the information to the MIR server 110.

The MIR server 110 searches for medical institutions corresponding to the selected medical category and transmits a list of medical institutions selected according to the search to the user terminal 200 (S1120).

The list of medical institutions is one or more medical institutions that may treat the user according to the user's symptom. Thereafter, a reply requesting a medical treatment appointment or a reply not requesting a medical treatment appointment may be received from the user terminal 200.

The list of selected medical institutions may include one medical subject or two or more medical subjects depending on the pre-visiting medical institution information.

Selection of a medical institution may be performed variously. For example:
i) The MIR server 110 may preferentially select a medical institution close to the user's residence or current location.
ii) The MIR server 110 may select a medical institution where the user has previously been treated.
iii) The MIR server 110 may preferentially select a medical institution with a high rank from the post-medical treatment information included in another user's healthcare record.

In addition to the above example, the MIR server 110 may select a medical institution in various manners.

The MIR server 110 receives medical treatment appointment information from the user terminal 200 that provides the list of medical institutions, and confirms the request for appointment at the medical institution from the user (S1121).

The user terminal 200 may select one of the list of medical institutions provided by the MIR server 110 to generate medical treatment appointment information.

The user terminal 200 may not select a medical institution from the list of medical institutions provided by the MIR server 110.

The user terminal 200 may generate medical treatment appointment information including information on one medical institution selected from the list of one or more medical institutions using an application.

The user terminal 200 may generate medical treatment appointment information by further including specific information, such as a date and time when medical treatment is available for a medical treatment appointment using the application.

The MIR server 110 requests a medical treatment appointment from the medical institution terminal 300 according to the confirmed medical treatment appointment information, receives a reply accepting the request, and completes the medical treatment appointment (S1140).

In an implementation of the present disclosure, the medical institution terminal 300 provides a reply corresponding to completion of the medical treatment appointment to the MIR server 110 in response to the medical treatment appointment request from the MIR server 110.

In another implementation of the present disclosure, the medical institution terminal 300 provides a reply corresponding to completion of the medical treatment appointment to the user terminal 200 in response to the medical treatment appointment request from the MIR server 110.

The user may be treated by a medical professional at the corresponding medical institution according to the medical treatment appointment (S1200).

In an implementation of the present disclosure, a user may visit the medical institution where a medical treatment appointment has been made, and may be treated.

In another implementation of the present disclosure, a medical professional from a medical institution where a medical treatment appointment has been made may visit a location for the user and provide medical treatment to the user.

In another implementation of the present disclosure, the user may receive remote medical treatment using the user terminal 200 and the medical institution terminal 300 of a medical institution. At this time, the user and the medical institution may perform remote medical treatment using a terminal other than the user terminal 200 and/or the medical institution terminal 300.

The user inputs medical examination and treatment information according to medical treatment by a medical professional at a medical institution into the user terminal 200 and provides the information to the MIR server 110 (S1300).

The medical examination and treatment information includes medical treatment information provided by a medical professional according to the user's treatment. The medical treatment information includes information, such as the name of a disease, therapeutic drugs, and treatment method.

In an implementation of the present disclosure, the medical treatment information provided by a medical professional to a user may be provided orally.

In another implementation of the present disclosure, the medical treatment information provided by a medical professional to a user may be provided in writing.

In another implementation of the present disclosure, the medical treatment information provided by a medical professional to a user may be provided through electronic media, such as electronic documents/images/videos/sound.

In another implementation of the present disclosure, the medical treatment information provided by a medical professional to a user may be provided using any one or more of the oral, written, and electronic media.

The user may input the medical examination and treatment information into the user terminal 200 using medical treatment information provided by a medical professional at a medical institution and provide the information to the MIR server 110.

In an implementation of the present disclosure, the medical examination and treatment information may be input to the user terminal 200 and provided to the MIR server 110.

In another implementation of the present disclosure, the medical examination and treatment information may be input to the user terminal 200, and may further include medical treatment information provided from the medical institution terminal 300 to the user terminal 200 and provided to the MIR server 110.

In another implementation of the present disclosure, the medical examination and treatment information may include medical treatment information provided from the medical institution terminal 300 to the MIR server 110.

The user performs treatment using therapeutic drugs and/or treatment methods that may be included in the medical treatment information according to the medical treatment at the medical institution, inputs post-medical treatment information including treatment results using the user terminal 200, and provides the same to the MIR server 110 (S 1400).

The post-medical treatment information input from the user terminal 200 may be input more than once for a predetermined period of time. Medical treatment for the user's disease may be performed once or more, and accordingly, the medical examination and treatment information and/or post-medical treatment information may be input multiple times and provided to the MIR server 110.

The post-medical treatment information includes therapeutic drugs and/or treatment methods administered and/or treated during a treatment period. The user may include information on auxiliary actions (diet, exercise, fasting, etc.) in the post-medical treatment information.

When input of the post-medical treatment information is completed, the user terminal 200 may input the status of treatment completion and provides the status to the MIR server 110.

In an implementation of the present disclosure, while inputting final post-medical treatment information, the user may additionally input information indicating that treatment for the corresponding disease has been completed and provide the final post-medical treatment information to the MIR server 110.

In another implementation of the present disclosure, the user inputs one or more pieces of post-medical treatment information. After the user inputs the post-medical treatment information, when the treatment is completed, the user inputs information indicating that the treatment has been completed into the user terminal 200 and provides the information to the MIR server 110.

When treatment completion information is provided from the user terminal 200, the MIR server 110 generates an individual-directed information record sheet using the stored healthcare record of the user (S1600).

The individual-directed information record sheet generated by the MIR server 110 may include media files, such as text, electronic documents, and images/videos/audio.

The MIR server 110 may generate a single, individual-directed information record sheet corresponding to the user's symptom.

In an implementation of the present disclosure, when the user's symptom occurs periodically within a predetermined period of time, the MIR server 110 may generate a single individual-directed information record sheet for the corresponding periodic symptom.

In another implementation of the present disclosure, when the user's symptom occurs periodically within a predetermined period of time, the MIR server 110 may generate a plurality of individually directed information record sheets for each periodic symptom.

The MIR server 110 of the present disclosure may determine whether to include the user's PHR information before generating an individual-directed information record sheet (S1500). PHR information is the user's PHR and is stored in government and/or public institutions.

If an individual-directed information record sheet is generated without PHR information in step S1500, the MIR server 110 may perform step S1600 to generate an individual-directed information record sheet.

In step S1500, when generating an individual-directed information record sheet including PHR information, the MIR server 110 provides the user's information to the government/public institution terminal 400 and requests the user's PHR information (S1510).

The government/public institution terminal 400 provides the stored user's PHR information to the MIR server 110 in response to the request for the user's PHR information (S1520).

The government/public institution terminal 400 may check the user information provided by the MIR server 110 before providing the user's PHR information. The government/public institution terminal 400 may request approval for sharing the PHR information from the user terminal 200 according to the confirmed user information.

The user terminal 200 inputs approval or rejection in response to the approval request and provides the input information to the government/public institution terminal 400.

The government/public institution terminal 400 may provide or reject the user's PHR information according to the approval or rejection provided from the user terminal 200.

If the MIR server 110 receives the user's PHR information, it generates an individual-directed information record sheet including the received PHR information (S1610).

In an implementation of the present disclosure, the MIR server 110 may generate an individual-directed information record sheet including information provided by the user terminal 200.

In another implementation of the present disclosure, the MIR server 110 may generate an individual-directed information record sheet including information provided from the user terminal 200 and the medical institution terminal 300.

In another implementation of the present disclosure, the MIR server 110 may generate an individual-directed information record sheet including information provided from the user terminal 200, the medical institution terminal 300, and the government/public institution terminal 400.

The MIR server 110 of the present disclosure generates an individual-directed information record sheet including PHR information or an individual-directed information record sheet not including PHR information and then stores the same in the search DB (S1700).

The search DB may store a plurality of individual-directed information record sheets generated by the MIR server 110 using healthcare records provided by a plurality of users. The search DB may provide individual-directed information record sheets retrieved in response to search requests for the stored individual-directed information record sheets from user terminals.

In an implementation of the present disclosure, the user terminal 200, the medical institution terminal 300, the other user terminal 500, and the other medical institution terminal 600 may be mobile terminals, and each terminal may be plural. Users and medical institutions that are subjects of usage of each terminal are registered as members of the individual-directed information record sheet generating system 100, and each terminal may be permitted to access the MIR server 110 and the search DB 120.

In another implementation of the present disclosure, the user terminal 200, the medical institution terminal 300, the other user terminal 500, the other medical institution terminal 600, and a general user terminal may be mobile terminals, and each terminal may be plural. Users, medical institutions, and general users who are the subjects of usage of each terminal, may be registered as members or non-members of the individual-directed information record sheet generating system 100. In the case of a non-member terminal, an individual-directed information record sheet may be searched and read from the search DB 120 by paying a certain fee.

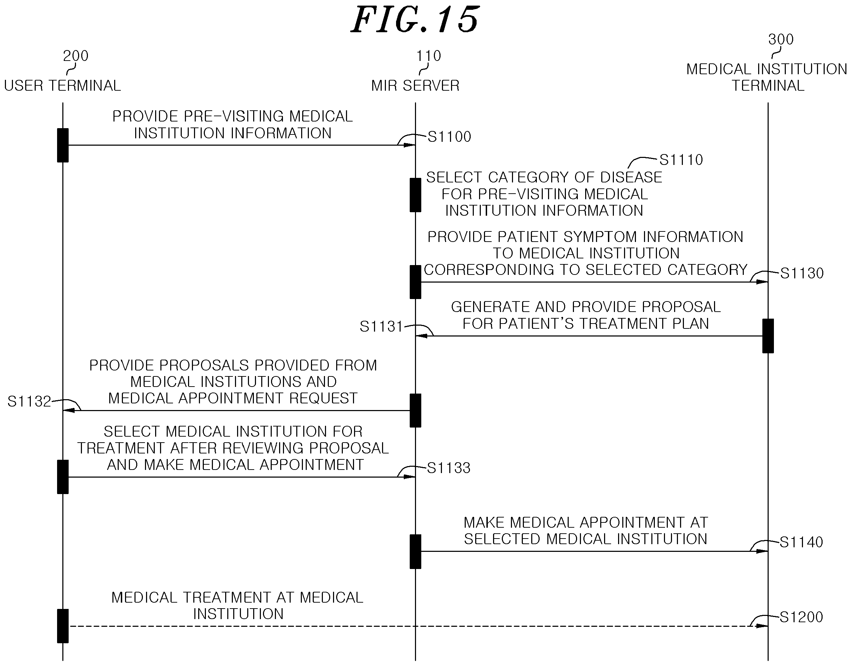

FIG. 15 is a flowchart illustrating a method of suggesting treatment at a medical institution according to a user's symptom in an individual-directed information record sheet generating system according to another implementation of the present disclosure. FIG. 6 is another implementation of the method for making a medical treatment appointment at a medical institution described in FIG. 4, which will be described as follows with reference thereto.

The user terminal 200 provides the input pre-visiting medical institution information to the MIR server 110 (S 1100).

The MIR server 110 may select a medical category by analyzing the received pre-visiting medical institution information (S1110). A method by which the MIR server 110 selects a medical category may be referred to in FIG. 4.

In an implementation of the present disclosure, the MIR server 110 may select a medical category as in step S1110 above.

In another implementation of the present disclosure, the MIR server 110 may consider selecting a medical category as in step S1110.

The MIR server 110 selects a medical institution corresponding to the selected medical category and provides the pre-visiting medical institution information to the selected medical institution (S1130).

The selected medical institutions may be plural, and the method for selecting a medical institution is described in FIG. 4.

In an implementation of the present disclosure, the MIR server 110 may select a medical institution corresponding to the medical category in step S1130.

In another implementation of the present disclosure, if a medical category is not selected in step S1130, the MIR server 110 may provide the pre-visiting medical institution information of the user to all medical institutions. In this case, among the medical institution terminals 300 that have received the pre-visiting medical institution information, a medical institution terminal that wants to provide medical treatment and/or treat the user may generate a proposal as in step S1131 and provide the same to the MIR server 110.

It is preferable that personal information is excluded from the pre-visiting medical institution information of the user provided by the MIR server 110.

For example, the pre-visiting medical institution information may exclude personal information, such as a name, a resident registration number, a phone number, and an address that may specify the user. In addition, in images and/or video materials attached to explain the user's symptom and symptom site, the user's face may be deleted, mosaiced, or blurred so that the user may not be specified.

If the attached image and/or video data is the user's face, the image and/or video data may be provided to a medical institution only with the user's consent.

The medical institution terminal 400, which has received the pre-visiting medical institution information, may input a proposal for treating the user's symptom and provide the same to the healthcare record providing server110 (S1131).

There may be multiple medical institutions that receive the pre-visiting medical institution information, and the medical institution prepares a proposal corresponding to the received pre-visiting medical institution information.

The proposal may include one or more information including treatment methods for treating the user's symptom and/or disease, treatment period, description of medical technology for treatment, treatment cost, etc.

In other words, the medical institution may prepare information necessary for treating the user based on the pre-visiting medical institution information and provide the same to the user. The user may review the proposals provided by the medical institutions that may treat the symptom, and select a medical institution for treatment.

In an implementation of the present disclosure, the user may select a proposal provided by any one medical institution among the received proposals.

In another implementation of the present disclosure, the user may select proposals provided by two or more medical institutions among the received proposals.

In another implementation of the present disclosure, the user may not select any of the received proposals.

The MIR server 110 provides the user terminal 200 with a list of medical institutions including proposals received from terminals of one or more medical institutions (S 1132).

The MIR server 110 may receive proposals from terminals of a plurality of medical institutions and may selectively provide the received proposals to the user terminal 200. For example, if there is an item missing from the contents of the proposals or if treatment costs are significantly different from the treatment costs written in other proposals, the proposal may be determined to be abnormal and the MIR server 110 may automatically exclude the proposal, when the proposals are provided to the user terminal 200.

While providing the proposal, the MIR server 110 may also provide a medical treatment appointment request including a list of medical institutions in which a medical treatment appointment may be made to the medical institution corresponding to the provided proposal.

The user terminal 200 may display one or more proposals provided by the MIR server 110 and provide medical institution and medical treatment appointment information selectively input by the user for treatment to the MIR server 110 (S1133).

The user terminal 200 may generate medical treatment appointment information for treatment at one or more medical institutions selected from the list of medical institutions provided by the MIR server 110.

The user terminal 200 may generate medical treatment appointment information including information on selecting one medical institution from the list of one or more medical institutions using an application.

The user terminal 200 may generate medical treatment appointment information by further including specific information, such as a date and time when medical treatment is available for a medical treatment appointment using the application.

Thereafter, the MIR server 110 requests a medical treatment appointment from the medical institution terminal 300 according to the confirmed medical treatment appointment information, receives a reply accepting the request, and completes the medical treatment appointment (S1140).

In an implementation of the present disclosure, the medical institution terminal 300 provides a reply corresponding to completion of the medical treatment appointment to the MIR server 110 in response to the medical treatment appointment request from the MIR server 110.

In another implementation of the present disclosure, the medical institution terminal 300 provides a reply corresponding to completion of the medical treatment appointment to the user terminal 200 in response to the medical treatment appointment request from the MIR server 110.

The user may be treated by a medical professional at the corresponding medical institution according to the medical treatment appointment (S1200).

Specific implementation of step S1200 may refer to step S1200 described above with reference to FIG. 13.

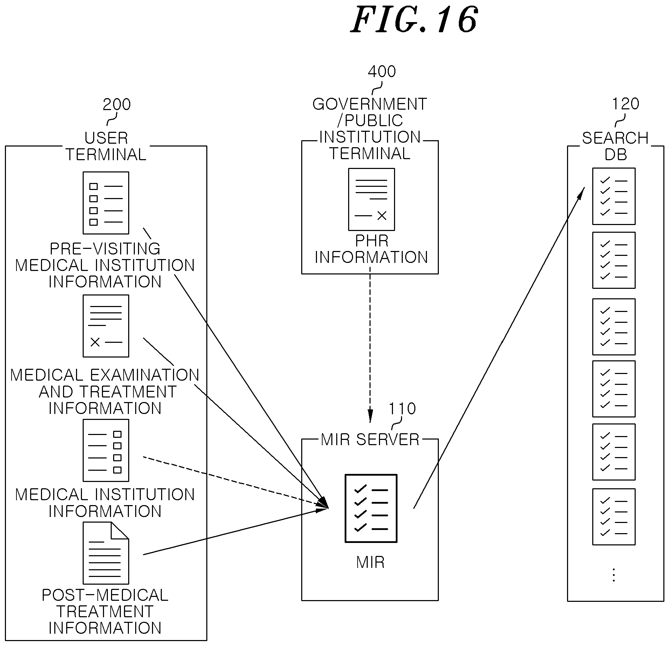

FIG. 16 is an exemplary diagram illustrating the creation and use of an individual-directed information record sheet generated according to another embodiment of the present disclosure. The individual-directed information record sheet is described as follows with reference to FIG. 16.

In an implementation of the present disclosure, the MIR server 110 may generate an individual-directed information record sheet in which a healthcare record provided from the user terminal 200 includes pre-visiting medical institution information, medical examination and treatment information, and post-medical treatment information.

In another implementation of the present disclosure, the MIR server 110 may generate an individual-directed information record sheet including the pre-visiting medical institution information, medical examination and treatment information, and post-medical treatment information provided from the user terminal 200 and further including medical institution information as necessary.

In another implementation of the present disclosure, the MIR server 110 may generate an individual-directed information record sheet including pre-visiting medical institution information, medical examination and treatment information, and post-medical treatment information provided from the user terminal 200 and further including user's PHR information provided from a government/public institution terminal 400 as necessary.

In another implementation of the present disclosure, the MIR server 110 may generate an individual-directed information record sheet including pre-visiting medical institution information, medical examination and treatment information, and post-medical treatment information provided from the user terminal 200 and further including medical institution information and user's PHR information provided from a government/public institution terminal 400 as necessary.

It is preferable that the medical examination and treatment information, medical institution information, and post-medical treatment information provided in order to generate an individual-directed information record sheet in the MIR server 110 are provided from the user terminal 200 but may be additionally provided from the medical institution terminal 300 that treated the user as necessary.

The MIR server 110 stores the individual-directed information record sheet generated as in the above implementations in the search DB 120. The search DB 120 may search for and provide an individual-directed information record sheet corresponding to a search request from among a plurality of stored individual-directed information record sheets.

Terminals including the user terminal 200, the medical institution terminal 300, the government/public institution terminal 400, another user terminal 500, and another medical institution terminal 600 may provide information by applying security technology and/or encryption technology.

In an implementation of the present disclosure, the individual-directed information record sheet generating system 100 and terminals may be implemented by applying the highest level of security technology and/or encryption technology known to those skilled in the art to which the present disclosure pertains.

In another implementation of the present disclosure, the individual-directed information record sheet generating system 100 and terminals may be implemented by applying a normal level of security technology and/or encryption technology known to those skilled in the art to which the present disclosure pertains.

In another implementation of the present disclosure, the individual-directed information record sheet generating system 100 and terminals may be implemented by applying the minimum level of security technology and/or encryption technology known to those skilled in the art to which the present disclosure pertains.

According to one aspect of the present disclosure, an individual-directed information record sheet generating system for generating an individual-directed information record sheet by a MIR server may be provided in order to generate an individual-directed information record sheet by a MIR server.

According to one aspect of the present disclosure, a computing device includes a memory configured to store at least one instruction; and a processor, in order to generate a membershiped information record sheet by an membership-led information record (MIR) server, wherein the processor is configured to, by performing the at least one instruction, (a) receive, by a MIR server, log on information from a terminal of a membershiped information provider in which a MIR application is run and validating the membershiped information provider, wherein the membershiped information provider is registered as a member in the MIR application; (b) receive a category of interest corresponding to a topic of interest of the membershiped information provider; (c) provide a terminal environment-controlling instruction to the terminal of the membershiped information provider so that information on a topic of interest of the membershiped information provider is input, wherein the terminal environment-controlling instruction includes an information editing module, an information sharing-range setting module, and an information record sheet that is used in the terminal of the membershiped information provider; (d) receive time-series information, which is information on the topic of interest input through the MIR application, in a time-series manner, wherein the time-series information is generated by inputting information on the topic of interest at least twice at time intervals; and (e) generate MIR using the time series information, wherein the MIR is tracked by a tracking module of the MIR server.

Since each component described in one aspect of the present disclosure overlaps the method of generating an individual-directed information record sheet by a MIR server described above with reference to FIGS. 1 to 9, descriptions thereof are omitted.

According to one aspect of the present disclosure, in a computer-readable recording medium storing a computer program, the computer-readable recording medium, which generates a membershiped information record sheet by an membership-led information record (MIR) server, stores a program to (a) receive, by a MIR server, log on information from a terminal of a membershiped information provider in which a MIR application is run and validate the membershiped information provider, wherein the membershiped information provider is registered as a member in the MIR application; (b) receive a category of interest corresponding to a topic of interest of the membershiped information provider; (c) provide a terminal environment-controlling instruction to the terminal of the membershiped information provider so that information on a topic of interest of the membershiped information provider is input, wherein the terminal environment-controlling instruction includes an information editing module, an information sharing-range setting module, and an information record sheet that is used in the terminal of the membershiped information provider; (d) receive time-series information, which is information on the topic of interest input through the MIR application, in a time-series manner, wherein the time-series information is generated by inputting information on the topic of interest at least twice at time intervals; and (e) generate MIR using the time series information, wherein the MIR is tracked by a tracking module of the MIR server.

Since each component described in one aspect of the present disclosure overlaps the method of generating an individual-directed information record sheet by a MIR server described above with reference to FIGS. 1 to 9, descriptions thereof are omitted.

According to one aspect of the present disclosure, in a computer program stored in a computer-readable recording medium, the computer program generating a membershiped information record sheet by an membership-led information record (MIR) server is programmed to perform (a) receiving, by a MIR server, log on information from a terminal of a membershiped information provider in which a MIR application is run and validating the membershiped information provider, wherein the membershiped information provider is registered as a member in the MIR application; (b) receiving a category of interest corresponding to a topic of interest of the membershiped information provider; (c) providing a terminal environment-controlling instruction to the terminal of the membershiped information provider so that information on a topic of interest of the membershiped information provider is input, wherein the terminal environment-controlling instruction includes an information editing module, an information sharing-range setting module, and an information record sheet that is used in the terminal of the membershiped information provider; (d) receiving time-series information, which is information on the topic of interest input through the MIR application, in a time-series manner, wherein the time-series information is generated by inputting information on the topic of interest at least twice at time intervals; and (e) generating MIR using the time series information, wherein the MIR is tracked by a tracking module of the MIR server.

Since each component described in one aspect of the present disclosure overlaps the method of generating an individual-directed information record sheet by a MIR server described above with reference to FIGS. 1 to 9, descriptions thereof are omitted.

According to one aspect of the present disclosure, a method of generating an individual-directed information record sheet including the following steps in a system for generating an individual-directed information record sheet includes: a process in which pre-visiting medical institution information is input and stored through a terminal of a membershiped information provider; a process in which, after a medical institution treats the user, medical examination and treatment information of the user is input and stored; and a process in which post-medical treatment information regarding the medical treatment is input and stored through a terminal of the membershiped information provider.

Since each component described in one aspect of the present disclosure overlaps the method of generating an individual-directed information record sheet by a MIR server described above with reference to FIGS. 10 to 16, descriptions thereof are omitted.

According to one aspect of the present disclosure, an individual-directed information record sheet generating system including the following to generate an individual-directed information record sheet includes an individual-directed information record sheet generating server configured to receive a healthcare record from a terminal of a membershiped information provider and generate an individual-directed information record sheet, wherein the healthcare record input from the terminal of the membershiped information provider includes pre-visiting medical institution information, medical examination and treatment information, and post-medical treatment information; and a search DB configured to allow a plurality of individual-directed information record sheets including the individual-directed information record sheet to be searched, wherein the search DB stores a plurality of individual-directed information record sheets generated by the individual-directed information record sheet generating server and provides an individual-directed information record sheet corresponding to search requests from other terminals.

Since each component described in one aspect of the present disclosure overlaps the method of generating an individual-directed information record sheet by a MIR server described above with reference to FIGS. 10 to 16, descriptions thereof are omitted.

According to one aspect of the present disclosure, in a computer-readable recording medium storing a computer program, the computer program generating an individual-directed information record sheet by a MIR server is stored in a recording medium to perform a step in which pre-visiting medical institution information of a user is input and stored through a terminal of a membershiped information provider; a step in which, after a medical institution treats the user, medical examination and treatment information is input and stored, wherein the medical examination and treatment information further includes medical treatment information provided from the medical institution; a step in which post-medical treatment information regarding the medical treatment is input and stored through the terminal of the membershiped information provider, wherein the post-medical treatment information is input at least once; and a step in which the input information is generated as an individual-directed information record sheet.

Since each component described in one aspect of the present disclosure overlaps the method of generating an individual-directed information record sheet by a MIR server described above with reference to FIGS. 10 to 16, descriptions thereof are omitted.

According to one aspect of the present disclosure, in a computer program stored in a computer-readable recording medium, the computer program that generates an individual-directed information record sheet by a MIR server is programmed to perform a step in which pre-visiting medical institution information of a user is input and stored through a terminal of a membershiped information provider; a step in which, after a medical institution treats the user, medical examination and treatment information is input and stored, wherein the medical examination and treatment information further includes medical treatment information provided from the medical institution; a step in which post-medical treatment information regarding the medical treatment is input and stored through the terminal of the membershiped information provider, wherein the post-medical treatment information is input at least once; and a step in which the input information is generated as an individual-directed information record sheet.

Since each component described in one aspect of the present disclosure overlaps the method of generating an individual-directed information record sheet by a MIR server described above with reference to FIGS. 10 to 16, descriptions thereof are omitted.

The above description is merely exemplary description of the technical scope of the present disclosure, and it will be understood by those skilled in the art that various changes and modifications can be made without departing from original characteristics of the present disclosure. Therefore, the embodiments disclosed in the present disclosure are intended to explain, not to limit, the technical scope of the present disclosure, and the technical scope of the present disclosure is not limited by the embodiments. The protection scope of the present disclosure should be interpreted based on the following claims and it should be appreciated that all technical scopes included within a range equivalent thereto are included in the protection scope of the present disclosure.

## Claims

1. A method of generating a member information record sheet to be performed by a membership-led information record (MIR) server, the method comprising:
(a) receiving, by the MIR server, log on information from a terminal of a membershiped information provider in which a MIR application is executed and validating the membershiped information provider, wherein the membershiped information provider is registered as a member in the MIR application;
(b) receiving a category of interest corresponding to a topic of interest of the membershiped information provider;
(c) providing a terminal environment-controlling instruction to the terminal of the membershiped information provider to input information on the topic of interest of the membershiped information provider, wherein the terminal environment-controlling instruction includes an information editing module, an information sharing-range setting module, and an information record sheet that is used in the terminal of the membershiped information provider;
(d) receiving time-series information, which is information on the topic of interest input through the MIR application, in a time-series manner, wherein the time-series information is generated by inputting information on the topic of interest at least twice at time intervals; and
(e) generating MIR using the time series information, wherein the MIR is tracked by a tracking module of the MIR server.

2. The method of claim 1, wherein the MIR is generated to include additional information that is further input one or more times at the time intervals after an intermediate MIR is generated using the input information on the topic of interest.

3. The method of claim 2, wherein the MIR is generated by:
(i) incorporating the additional information into the intermediate MIR;
(ii) modifying the intermediate MIR with the additional information;
(iii) generating another intermediate MIR using the additional information separate from the intermediate MIR and then using two intermediate MIRs to generate the MIR; and/or
(iv) performing a combination of the (i) and the (ii).

4. The method of claim 1, wherein, in the tracking, the MIR server provides development history from a perspective of the time series manner.

5. The method of claim 4, wherein the development history includes at least one history selected from the group consisting of a reception history of the time-series information, a change history of information on the topic of interest, and a creation history of the MIR.

6. The method of claim 1, wherein the MIR is an edited information record.

7. The method of claim 6, wherein the edited information record includes a plurality of MIR editions in which edition is assigned to each of the MIR editions.

8. The method of claim 6, wherein, in the tracking, the MIR server provides development history in terms of the edition, and the development history includes the edition assignment history.

9. The method of claim 7, wherein the MIR edition includes meta information on the edition.

10. The method of claim 7, wherein the MIR includes two or more MIR editions, and the two or more MIR editions are selected by a MIR server or the membershiped information provider.

11. The method of claim 7, wherein the information on the topic of interest is generated as a MIR edition by assigning an edition according to a point in time at which the information on the topic of interest is received in the time-series manner.

12. The method of claim 7, wherein, when additional information on the topic of interest is input after creation of the MIR edition, the MIR server is configured to
(i) generate the MIR edition including the additional information on the topic of interest;
(ii) automatically generate a new MIR edition; or
(iii) generate a new MIR edition in response to a request of the membershiped information provider.

13. The method of claim 6, wherein the edition is assigned in plurality, and the plurality of assigned editions are structured hierarchically.

14. The method of claim 1, wherein the MIR server provides the membershiped information provider with the authority to edit the MIR and determine an information sharing range of the MIR.

15. The method of claim 14, wherein the editing is performed using the information editing module, and the determining of the range of sharing information is performed using the information sharing range setting module.

16. The method of claim 1, wherein the category of interest includes a hierarchical information level having multiple levels in the topic of interest.

17. The method of claim 16, wherein the MIR server receives the category of interest at a lowest level of the hierarchical information level.

18. The method of claim 1, wherein the category of interest includes at least one selected from a group including at least two or more from the group including healthcare, art, research, cooking, pets, animal husbandry, plant cultivation, travel, religion, and sports.

19. The method of claim 7, wherein, when the category of interest is healthcare, the MIR is a healthcare record of an user selected from the membershiped information provider and a patient managed by the membershiped information provider; and the MIR includes:
(i) a MIR edition including, as pre-visiting medical institution information, information on symptoms of a disease, disorder, or condition;
(ii) a MIR edition including, as medical examination and treatment information, information including a result of medical examination, treatment, surgery, prescription medication and/or therapy done by a medical professional; and
(iii) a MIR edition including, as post-medical treatment information, information on the user's progress after treatment.

20. The method of claim 19, wherein the pre-visiting medical institution information, the medical examination and treatment information, and the post-medical treatment information are types of time-series information,
the time-series information is generated by inputting information on the topic of interest three or more times at time intervals,
one of the types of information input three or more times includes the pre-visiting medical institution information, another includes the medical examination and treatment information, and the other includes post-medical treatment information.

21. The method of claim 19, wherein the MIR is generated by
receiving and storing the pre-visiting medical institution information through the terminal of the membershiped information provider;
receiving and storing the medical examination and treatment information of the user after treating the user at a medical institution; and
receiving and storing the post-medical treatment information for the medical treatment through the terminal of the membershiped information provider.

22. The method of claim 21, further comprises, after generating the MIR, storing the MIR to be searched in a search DB to enable the MIR to be searchable.

23. The method of claim 22, wherein the MIR stored in the search DB is searchable on the terminal of the membershiped information provider and/or another user terminal.

24. The method of claim 22, wherein the MIR stored in the search DB is searchable on a medical institution terminal of the medical institution and/or a medical institution terminal of another medical institution.

25. The method of claim 22, wherein the search is performed on at least one information selected from the group consisting of pre-visiting medical institution information, the medical examination and treatment information, and post-medical treatment information.

26. The method of claim 21, wherein the pre-visiting medical institution information includes a health questionnaire.

27. The method of claim 26, wherein the health questionnaire is provided by the terminal of the membershiped information provider or the MIR server.

28. The method of claim 21, wherein the medical examination and treatment information is input through the terminal of the membershiped information provider.

29. The method of claim 21, wherein the medical examination and treatment information is further input through the medical institution terminal of the medical institution.

30. The method of claim 21, wherein the medical examination and treatment information include a disease name for the user's disease and further include therapeutic drug information and/or treatment method information.

31. The method of claim 21, further includes, after receiving and storing the medical examination and treatment information, receiving and storing a medical institution information that has treated the user.

32. The method of claim 31, wherein the medical institution information is input through the terminal of the membershiped information provider or the medical institution terminal of the medical institution.

33. The method of claim 21, further includes, after receiving and storing the pre-visiting medical institution information, selecting a medical category corresponding to the input pre-visiting medical institution information.

34. The method of claim 33, wherein the medical category includes a plurality of items in which the plurality of items are provided by sub-classifying one or more selected from the group consisting of a medical department, a type of symptom, a site of symptom and a type of disease, and wherein the medical category is selected from at least one of the plurality of items.

35. The method of claim 33, further includes, after the selecting the medical category, providing a list of medical institutions corresponding to the selected medical category to the terminal of the membershiped information provider and making an appointment with any one medical institution that is selected from the provided list of medical institutions by the terminal of the membershiped information provider..

36. The method of claim 21, wherein the medical examination and treatment information further include medical treatment information of the medical institution regarding the medical treatment.

37. The method of claim 21, wherein the post-medical treatment information is further input through the medical institution terminal of the medical institution.

38. The method of claim 21, wherein the MIR further includes a personal health record (PHR) stored in a government or public institution.

39. The method of claim 21, wherein the medical treatment includes the user's onsite medical treatment and/or the user's remote medical treatment.

40. The method of claim 7, wherein, when the category of interest is healthcare, the MIR is a healthcare record of an user selected from the membershiped information provider and a patient managed by the membershiped information provider; and the MIR includes:
(i) a MIR edition including, as pre-visiting medical institution information, information on symptoms of a disease, disorder, or condition; and
(ii) a MIR edition including, as post-medical treatment information, information on the user's progress after treatment.

41. The method of claim 40, wherein the pre-visiting medical institution information is generated by receiving information on the symptoms of the disease, disorder, or condition twice or more at time intervals, and
the post-medical treatment information is generated by receiving information on the progress after the treatment twice or more at time intervals.

42. The method of claim 1, wherein the terminal environment-controlling instruction is selectively provided in response to the received category of interest.

43. The method of claim 42, wherein the terminal environment-controlling instruction is selectively provided in response to information on the topic of interest input at the time intervals.

44. The method of claim 7, wherein the terminal environment-controlling instruction is provided in response to the received category of interest and the MIR edition.

45. The method of claim 1, wherein the information editing module is a control command and/or an application to utilize a text input module, an image generating module (or a drawing module), a touch input module, a camera, a microphone, a location recognition module (or a GPS module) and/or a sensor module for recording information in the terminal of the membershiped information provider.

46. The method of claim 1, wherein the information sharing setting module receives information to determine
(i) a range of a membershiped user in the MIR server to who the MIR is accessible; and/or
(ii) the MIR to be accessible to the membershiped user in the MIR server and/or a range of information in the MIR to be accessible to the membershiped user.

47. The method of claim 1, wherein an edition is assigned to at least two or more of the plurality of time-series information received in the time-series manner to generate a plurality of MIR editions, the plurality of MIR editions each includes meta information and the plurality of MIR editions are incorporated in the MIR.

48. The method of claim 7, wherein the MIR server selects the information record sheet depending on the information on the topic of interest input at the time intervals or the edition assigned to the MIR edition and then provides the selected information record sheet to the terminal of the membershiped information provider.

49. The method of claim 7, wherein the MIR server is configured to
(i) select at least one of a plurality of information record sheets and provide the selected at least one information record sheet to the terminal of the membershiped information provider so that the membershiped information provider may input standardized information corresponding to the information on the topic of interest input at the time intervals; or
(ii) select the at least one of the plurality of information record sheets and provide the selected at least one information record sheet to the terminal of the membershiped information provider so that the membershiped information provider may input standardized information corresponding to the edition.

50. A computing device for generating a membershiped information record sheet in a membership-led information record (MIR) server, the computing device comprising:
a memory configured to store at least one instruction; and
a processor configured to execute the one or more instructions stored in the memory, wherein the instructions, when executed by the processor, cause the processor to
(a) receive, by the MIR server, log on information from a terminal of a membershiped information provider in which a MIR application is executed and validating the membershiped information provider, wherein the membershiped information provider is registered as a member in the MIR application;
(b) receive a category of interest corresponding to a topic of interest of the membershiped information provider;
(c) provide a terminal environment-controlling instruction to the terminal of the membershiped information provider to input information on the topic of interest of the membershiped information provider, wherein the terminal environment-controlling instruction includes an information editing module, an information sharing-range setting module, and an information record sheet that is used in the terminal of the membershiped information provider;
(d) receive time-series information, which is information on the topic of interest input through the MIR application, in a time-series manner, wherein the time-series information is generated by inputting information on the topic of interest at least twice at time intervals; and
(e) generate MIR using the time series information, wherein the MIR is tracked by a tracking module of the MIR server.

51. A non-transitory computer-readable storage medium storing a computer executable instructions, wherein the instructions, when executed by a processor, cause the processor to perform a method for generating a membershiped information record sheet in a membership-led information record (MIR) server, the method comprising:
(a) receiving, by the MIR server, log on information from a terminal of a membershiped information provider in which a MIR application is run and validate the membershiped information provider, wherein the membershiped information provider is registered as a member in the MIR application;
(b) receiving a category of interest corresponding to a topic of interest of the membershiped information provider;
(c) providing a terminal environment-controlling instruction to the terminal of the membershiped information provider to input information on the topic of interest of the membershiped information provider, wherein the terminal environment-controlling instruction includes an information editing module, an information sharing-range setting module, and an information record sheet that is used in the terminal of the membershiped information provider;
(d) receiving time-series information, which is information on the topic of interest input through the MIR application, in a time-series manner, wherein the time-series information is generated by inputting information on the topic of interest at least twice at time intervals; and
(e) generating MIR using the time series information, wherein the MIR is tracked by a tracking module of the MIR server.

52. A computer program stored in a non-transitory computer-readable storage medium, wherein the computer program including a computer executable instructions , when executed by a processor, cause the processor to perform a method for generating a membershiped information record sheet in a membership-led information record (MIR) server, the method comprising:
(a) receiving, by the MIR server, log on information from a terminal of a membershiped information provider in which a MIR application is executed and validating the membershiped information provider, wherein the membershiped information provider is registered as a member in the MIR application;
(b) receiving a category of interest corresponding to a topic of interest of the membershiped information provider;
(c) providing a terminal environment-controlling instruction to the terminal of the membershiped information provider to input information on the topic of interest of the membershiped information provider, wherein the terminal environment-controlling instruction includes an information editing module, an information sharing-range setting module, and an information record sheet that is used in the terminal of the membershiped information provider;
(d) receiving time-series information, which is information on the topic of interest input through the MIR application, in a time-series manner, wherein the time-series information is generated by inputting information on the topic of interest at least twice at time intervals; and
(e) generating MIR using the time series information, wherein the MIR is tracked by a tracking module of the MIR server.
